(19)

Europäisches Patentamt

European Patent Office

Office européen des brevets

(11) **EP 2 989 578 B1**

(12) **EUROPEAN PATENT SPECIFICATION**

(45) Date of publication and mention of the grant of the patent:
**29.03.2023 Bulletin 2023/13**

(21) Application number: **14718979.9**

(22) Date of filing: **22.04.2014**

(51) International Patent Classification (IPC):
***G16B 5/00*** *(2019.01)*

(52) Cooperative Patent Classification (CPC):
**G16B 5/00**

(86) International application number:
**PCT/EP2014/058159**

(87) International publication number:
**WO 2014/173912 (30.10.2014 Gazette 2014/44)**

(54) **SYSTEMS AND METHODS FOR USING MECHANISTIC NETWORK MODELS IN SYSTEMS TOXICOLOGY**

SYSTEME UND VERFAHREN ZUR VERWENDUNG MECHANISTISCHER NETZWERKMODELLE IN SYSTEMTOXIKOLOGIE

SYSTÈMES ET MÉTHODES D'UTILISATION DE MODÈLES DE RÉSEAUX MÉCANISTES ASSOCIÉS À LA TOXICOLOGIE DES SYSTÈMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2013 US 201361815188 P**

(43) Date of publication of application:
**02.03.2016 Bulletin 2016/09**

(73) Proprietor: **Philip Morris Products S.A. 2000 Neuchâtel (CH)**

(72) Inventor: **MARTIN, Florian 2034 Peseux (CH)**

(74) Representative: **Ponder, William Anthony John et al Reddie & Grose LLP The White Chapel Building 10 Whitechapel High Street London E1 8QS (GB)**

(56) References cited:
**WO-A1-2012/168481    WO-A2-2013/034300**

- **JULIA HOENG ET AL: "A network-based approach to quantifying the impact of biologically active substances", DRUG DISCOVERY TODAY, vol. 17, no. 9-10, 1 May 2012 (2012-05-01) , pages 413-418, XP055071052, ISSN: 1359-6446, DOI: 10.1016/j.drudis.2011.11.008**
- **Anonymous: "Resampling (statistics) - Wikipedia, the free encyclopedia", , 21 April 2013 (2013-04-21), XP055130348, Retrieved from the Internet: URL:http://en.wikipedia.org/w/index.php?ti tle=Resampling_(statistics)&oldid=55141656 1#Permutation_tests [retrieved on 2014-07-18]**
- **M. J. HERRGARD: "Reconciling Gene Expression Data With Known Genome-Scale Regulatory Network Structures", GENOME RESEARCH, vol. 13, no. 11, 1 November 2003 (2003-11-01), pages 2423-2434, XP055066721, ISSN: 1088-9051, DOI: 10.1101/gr.1330003**
- **Anonymous: "Dot product - Wikipedia, the free encyclopedia", , 18 April 2013 (2013-04-18), XP055130381, Retrieved from the Internet: URL:http://en.wikipedia.org/w/index.php?ti tle=Dot_product&oldid=551038540 [retrieved on 2014-07-21]**

EP 2 989 578 B1

## Description

## BACKGROUND

The human body is constantly perturbed by exposure to potentially harmful agents that can pose severe health risks in the long-term. Exposure to these agents can compromise the normal functioning of biological mechanisms internal to the human body. To understand and quantify the effect that these perturbations have on the human body, researchers study the mechanism by which biological systems respond to exposure to agents. Some groups have extensively utilized *in vivo* animal testing methods. However, animal testing methods are not always sufficient because there is doubt as to their reliability and relevance. Numerous differences exist in the physiology of different animals. Therefore, different species may respond differently to exposure to an agent. Accordingly, there is doubt as to whether responses obtained from animal testing may be extrapolated to human biology. Other methods include assessing risk through clinical studies of human volunteers. But these risk assessments are performed *a posteriori* and, because diseases may take decades to manifest, these assessments may not be sufficient to elucidate mechanisms that link harmful substances to disease. Yet other methods include *in vitro* experiments. Although, *in vitro* cell and tissue-based methods have received general acceptance as full or partial replacement methods for their animal-based counterparts, these methods have limited value. Because *in vitro* methods are focused on specific aspects of cells and tissues mechanisms; they do not always take into account the complex interactions that occur in the overall biological system.

In the last decade, high-throughput measurements of nucleic acid, protein and metabolite levels in conjunction with traditional dose-dependent efficacy and toxicity assays, have emerged as a means for elucidating mechanisms of action of many biological processes. Researchers have attempted to combine information from these disparate measurements with knowledge about biological pathways from the scientific literature to assemble meaningful biological models. To this end, researchers have begun using mathematical and computational techniques that can mine large quantities of data, such as clustering and statistical methods, to identify possible biological mechanisms of action.

Previous work has also explored the importance of uncovering a characteristic signature of gene expression changes that results from one or more perturbations to a biological process, and the subsequent scoring of the presence of that signature in additional data sets as a measure of the specific activity amplitude of that process. Most work in this regard has involved identifying and scoring signatures that are correlated with a disease phenotype. These phenotype-derived signatures provide significant classification power, but lack a mechanistic or causal relationship between a single specific perturbation and the signature. Consequently, these signatures may represent multiple distinct unknown perturbations that, by often unknown mechanism(s), lead to, or result from, the same disease phenotype.

One challenge lies in understanding how the activities of various individual biological entities in a biological system enable the activation or suppression of different biological mechanisms. Because an individual entity, such as a gene, may be involved in multiple biological processes (*e.g.*, inflammation and cell proliferation), measurement of the activity of the gene is not sufficient to identify the underlying biological process that triggers the activity.

Recently, there has been an increased focus in systems toxicology on approaches that emphasize understanding the biological impact of chemical exposures with increased mechanistic granularity at the whole genome level. A report by the US National Research Council Committee on Toxicity Testing and Assessment of Environmental Agents advocates for a shift away from toxicological assessment at the level of apical endpoints and towards understanding the signaling pathways perturbed by biologically active substances and identifying those that have the potential to cause adverse health effects in humans [Krewski, D. et al. (2011) New directions in toxicity testing. Annu Rev Public Health. 32, 161-78; Bhattacharya, S. et al. (2011) Toxicity testing in the 21 century: defining new risk assessment approaches based on perturbation of intracellular toxicity pathways. PLoS One. 6, e20887; Keller, D.A. et al. (2012) Identification and characterization of adverse effects in 21st century toxicology. Toxicological Sciences. 126, 291-297; National Research Council. Committee on Toxicity Testing Assessment of Environmental Agents (2007), Toxicity testing in the 21st century: A vision and a strategy. N.A. Press]

This paradigm shift is also seen in pharmacology, where a more detailed understanding of the disease mechanism is needed for optimal preclinical and clinical safety testing [Atterwill, C.K. and Wing, M.G. (2002) In vitro preclinical lead optimisation technologies (PLOTs) in pharmaceutical development. Toxicology letters. 127, 143-151]. New technologies allow large-scale data generation, and evaluation of these data can provide mechanistic and even quantitative insights into both desired as well as side effects. The interpretation of differentially expressed lists of molecules has proven inefficient at gaining meaningful information, and the focus has shifted to pathway analysis and network model development using data obtained from experimental systems treated with compounds of interest [Lee, E. et al. (2008) Inferring pathway activity toward precise disease classification. PLoS computational biology. 4, e1000217]. However, many widely used pathway databases appear inconsistent with respect to the actual interactions in their networks. This inconsistency can be partially attributed to the frequent absence of standardized vocabularies and lack of curation/knowledge of tissue- or cell type-specific signaling mechanisms.

In the past decade, the systems biology community has experienced an increase in computational approaches for data-driven network inferencing, which does not require prior literature knowledge, but builds networks based on Systems Response Profiles (SRP) [Hoeng, J. et al. (2012) A network-based approach to quantifying the impact of biologically active substances. Drug Discov Today. 17, 413-8]. A prominent example is the Connectivity Map gene expression compendium, which is obtained from cell lines treated with a large array of compounds to compare drug and gene effects as well as identify network communities related to drugs with similar expression profiles [Lamb, J. et al. (2006) The Connectivity Map: using gene-expression signatures to connect small molecules, genes, and disease. Science Signalling. 313, 1929]. Inference is possible with little prior information, and different algorithms [Lamb, J. et al. (2006) The Connectivity Map: using gene-expression signatures to connect small molecules, genes, and disease. Science Signalling. 313, 1929; Xiang, Y. et al. (2011) Divergence Weighted Independence Graphs for the Exploratory Analysis of Biological Expression Data. Journal of Health & Medical Informatics; Iorio, F. et al. (2010) Discovery of drug mode of action and drug repositioning from transcriptional responses. Proceedings of the National Academy of Sciences. 107, 14621-14626] can be readily applied to transcriptional or proteomics responses obtained from new compounds in different phases of the drug discovery pipeline. However, these methods are still compromised by assumptions, simplifications, and often limited experimental data sets [Lecca, P. and Priami, C. (2012) Biological network inference for drug discovery. Drug discovery today]. The DREAM (Dialogue on Reverse Engineering Assessment and Methods) initiative has assessed the performance of 29 gene-network-inference methods by crowd-sourcing and concluded that "reliable network inference from gene expression data remains an unsolved problem" [Marbach, D. et al. (2010) Revealing strengths and weaknesses of methods for gene network inference. Proceedings of the National Academy of Sciences. 107, 6286-6291]. Julia Hoeng et. Al. A network-based approach to quantifying the impact of biologically active substances. Drug Discovery Today. vol. 17, no. 9 to 10 1 May 2012. pp 413-418 discloses a computational method for deriving a biological impact factor from underlying system-wide data using defined causal biological network models as the substrate for data analysis. This approach does, however, not disclose taking into account an overlap of two computational networks used in the computation of the overall perturbation via a scalar product.

WO2013/034300 discloses a computerized method for quantifying the perturbation of a biological system based on measured activity data from a subset of the entities in the biological system. A score is derived that is representative of the magnitude and topological distribution of the response of the network to the perturbation

## SUMMARY

The invention is defined by the claims.

Described herein are systems and methods for using high-throughput molecular level measurements and causal biological network models as substrates for computational analysis. This integrated approach provides insights into the pathogenic mechanisms of diseases at the biological systems level as well as a quantitative assessment of the perturbations of various biological systems caused by exposure to an agent or a stimulus.

According to an implementation of the disclosure, a processor or a processing circuitry receives data from experiments in which a biological system is perturbed, as well as control data. The processor or processing circuitry generates system response profiles (SRPs), which are representations of the degree to which one or more entities within a biological system change in response to the presentation of an agent to the biological system. Then, a network model is selected as being relevant to the agent or a feature of interest. Causal relationships between the entities within the system are extracted using the SRPs and networks, thereby generating, refining, or extending a network model.

In particular, a Network Perturbation Amplitude (NPA) is calculated. The NPA is a quantitative measurement of the impact, or a quantification of a biological response to a perturbation or treatment represented by the SRPs in the context of the underlying relationships between the biological entities represented by the network. Then, the NPAs from different network models are combined to derive a Biological Impact Factor (BIF). The BIF can be correlated with other measurable endpoints or used to generate testable hypotheses that could provide new insight into mechanisms of disease onset or progression. The BIF is then decomposed into individual network models, thus allowing for the identification of the biological processes that most contribute to the impact.

In certain embodiments of the systems and methods described herein, a computerized method quantifies a perturbation of a biological system in response to an agent. The computerized method comprises receiving, at a processing circuitry, a set of treatment data corresponding to a response of the biological system to the agent, and receiving a set of control data corresponding to a response of the biological system not exposed to the agent. A first computational causal network model and a second computational causal network model that each represents a biological process in the biological system are identified. The processing circuitry computes, based on the set of treatment data and the set of control data, a first score for the first computational causal network model and a second score for the second computational causal network model. A factor is generated based on the first score, the second score, and an intersection between the first computational causal network model and the second computational causal network model, wherein the factor represents the perturbation of the biological system in response to the agent.

In certain embodiments, the biological system includes a plurality of biological entities, each biological entity interacting with at least one other of the biological entities, and the intersection between the first computational causal network model and the second computational causal network model includes an overlapping set of nodes representative of a subset of the plurality of biological entities.

In certain embodiments, the method further comprises determining a statistical significance of the first score to assess a specificity of the first score with respect to the first computational causal network model. Determining the statistical significance of the first score may comprise modifying one or more aspects of the first computational causal network model to obtain a set of test models, computing a set of test scores based on the set of test models, and comparing the first score to the set of test scores. The one or more aspects may include labels assigned to a set of nodes in the first computational causal network model. The one or more aspects may include edge connections between nodes in the first computational causal network model. In certain embodiments, the modifying is performed randomly N times, and one of the test scores is computed each time the modifying is performed. In certain embodiments, the first score is statistically significant when the first score exceeds a threshold percentage of the set of test scores.

In certain embodiments, each of the first computational causal network model and the second computational causal network model is representative of a biological mechanism in the biological system. In certain embodiments, the computing the first score comprises assessing a norm on a signed directed graph underlying the first computational causal network model. The assessing the norm may comprise assessing an adjacency matrix.

In certain embodiments, the generating the factor comprises computing a scalar product that accounts for the intersection between the first computational causal network model and the second computational causal network model, and adjusting at least one of the first score and the second score based on the scalar product. In certain embodiments, the scalar product cancels the intersection and represents contributions of orthogonal portions of the first computational causal network model and the second computational causal network model. In certain embodiments, the scalar product defines an orthogonal direct sum.

The computerized methods described herein may be implemented in a computerized system having one or more computing devices, each including one or more processors or processing circuitries. Generally, the computerized systems described herein may comprise one or more engines, which include a processing device or devices, such as a computer, microprocessor, logic device or other device or processor that is configured with hardware, firmware, and software to carry out one or more of the computerized methods described herein. In certain implementations, the computerized system includes a systems response profile engine, a network modeling engine, and a network scoring engine. The engines may be interconnected from time to time, and further connected from time to time to one or more databases, including a perturbations database, a measurables database, an experimental data database and a literature database. The computerized system described herein may include a distributed computerized system having one or more processors and engines that communicate through a network interface. Such an implementation may be appropriate for distributed computing over multiple communication systems.

## BRIEF DESCRIPTION OF THE DRAWINGS

Further features of the disclosure, its nature and various advantages, will be apparent upon consideration of the following detailed description, taken in conjunction with the accompanying drawings, in which like reference characters refer to like parts throughout, and in which:

FIG. 1A illustrates a network model describing tissue and cell-specific biological processes in the form of causal relationships between biological entities.

FIG. 1B illustrates a causal biological network model with backbone nodes and supporting nodes for indicating cause and effect relationships.

FIG. 1C illustrates a network model that captures biology in the nodes and causal relationships between the nodes.

FIG. 1D illustrates a network model representative of knowledge from molecular, cellular, and organ levels to an entire organism.

FIG. 2A illustrates network perturbation amplitude scores assessed by mathematical transformation of differentially expressed genes using a network model.

FIG. 2B illustrates *in vivo* bronchial brushing datasets that were scored against a xenobiotic metabolism network model.

FIG. 2C illustrates a comparison of human bronchial brushing datasets in the context of a xenobiotic metabolism network.

FIG. 3A is a diagram of *in vivo* bronchial brushing and *in vitro* culture of bronchial epithelial cells exposed to smoke.

FIG. 3B illustrates an assessment of three *in vivo* datasets and an *in vitro* dataset in the context of a xenobiotic metabolism network.

FIG. 3C shows individual comparisons of the *in vivo* datasets to the *in vitro* dataset in the context of a xenobiotic metabolism network model.

FIG. 4A shows a biological impact factor cone illustrating various networks and corresponding individual perturbation amplitudes, showing the contribution of the various mechanisms to the overall biological impact factor.

FIG. 4B shows relative biological impact factor data for smoke-exposed mouse lungs using a Pulmonary Inflammatory Processes Network (IPN), Cell Proliferation Network, Cell Stress Network and sub networks that constitute DNA damage, Autophagy, Cell death (apoptosis and necrosis) and Senescence network (DACS).

FIG. 4C illustrates starplots showing a decomposition of the overall relative BIF into its main mechanistic components (from cell proliferation to inflammation) for various treatment groups.

FIG. 4D is a graphical representation of Bronchoalveolar lavage fluid (BALF) cell counts from smoke-exposed mice.

FIG. 4E is a graphical representation of NPA calculations for cell type-specific subnetworks within the Pulmonary Inflammatory Processes Network (IPN) and corresponding measurements from BALF for macrophage activation.

FIG. 4F is a graphical representation of NPA calculations for cell type-specific subnetworks within the Pulmonary Inflammatory Processes Network (IPN) and corresponding measurements from BALF for epithelial proinflammatory signaling.

FIG. 5 is a block diagram of an exemplary computing device which may be used to implement any of the components in any of the computerized systems described herein.

FIGS. 6 - 7 are flow diagrams of illustrative processes for determining a statistical significance of an NPA score.

## DETAILED DESCRIPTION

Described herein are computational systems and methods for analyzing large-scale molecular level measurements in the context of causal biological network models which allow quantitative assessment of the magnitude of changes within a biological system when it is perturbed by an agent. The approaches described herein allow for a detailed mechanistic understanding of the impact that a given stimulus inflicts upon a biological system.

The total volume of pathway information has grown dramatically, with the number of online resources for pathways and molecular interactions increasing 70% from 190 in 2006 [Bader, G.D. Cary, M.P. and Sander, C. (2006) Pathguide: a pathway resource list. Nucleic Acids Research. 34, D504-D506] to 325 in 2010. This strongly indicates that the scientific community recognizes that pathways, and eventually networks, greatly facilitate the understanding of the effects that biologically active substances have on biological systems. Network biology provides a coherent framework for investigating the impact of exposures at the molecular, pathway, and process levels [Hasan, S. et al. (2012) Network analysis has diverse roles in drug discovery. Drug discovery today]. Drugs for many disease states may require multiple activities to be efficacious; thus, network biology may indeed hold clues to designing drugs that perturb biological networks rather than individual targets [Yıldırım, M.A. et al. (2007) Drug-target network. Nature Biotechnology. 25, 1119]. Moreover, network biology provides a platform to potentially understand side effects of drug candidates as well as predictions in polypharmacology [Hopkins, A.L. (2008) Network pharmacology: the next paradigm in drug discovery. Nature chemical biology. 4, 682-690].

The disclosure herein describes a network-based approach for employing high-throughput data and causal biological network models as the substrate for data analysis. According to an implementation of the disclosure, a processor receives data from experiments in which a biological system is perturbed, as well as control data. The processor may be a system response profile (SRP) engine, which generates SRPs. SRPs are representations of the degree to which one or more entities within a biological system change in response to the presentation of an agent to the biological system. Then, a processor such as a network modeling engine may provide one or more databases that contain(s) a plurality of network models, one of which is selected as being relevant to the agent or a feature of interest. The selection can be made on the basis of prior knowledge of the mechanisms underlying the biological functions of the system. The network modeling engine may extract causal relationships between the entities within the system using the SRPs and networks in the database, thereby generating, refining, or extending a network model. To identify the causal effects induced by the exposure, the impact of a stimulus is measured on every node in the one or more networks which relates to one or more features of a biological system.

Moreover, the network modeling engine computes a Network Perturbation Amplitude (NPA) score [Martin, F. et al. (2012) Assessment of network perturbation amplitude by applying high-throughput data to causal biological networks. BMC Syst Biol. 6, 54], which is a quantitative measurement of the impact. The term "score" is used herein generally to refer to a value or set of values which provide a quantitative measure of the magnitude of changes in a biological system. Such a score is computed by using any of various mathematical and computational algorithms known in the art and according to the methods disclosed herein, employing one or more datasets obtained from a sample or a subject. In particular, the NPA score is a quantification of a biological response to a perturbation or treatment represented by the SRPs in the context of the underlying relationships between the biological entities represented by the network. Then, the NPAs from different network models can be combined to derive a Biological Impact Factor (BIF). The BIF can be correlated with other measurable endpoints or controls, or used to generate testable hypotheses that could provide new insight into mechanisms of disease onset or progression.

The systems and methods of the present disclosure allow for robust comparison of responses in different experimental systems (transcriptional response in the examples) and enables for the identification of network-based biomarkers that are modulated in response to exposure to a particular agent or stimulus. Moreover, the network scores are correlated with other relevant endpoints from a given experiment. The systems and methods described herein are also applicable to pharmacology, where the understanding of disease mechanisms and adverse drug effects is important for the development of efficient and safe treatment options. In an example, the given stimulus is cigarette smoke (CS) or smoking-related diseases. However, one of ordinary skill in the art will understand that the systems and methods described herein can be applied to any substance or treatment in a relevant biological system context.

A biological system in the context of the present disclosure is an organism or a part of an organism, including functional parts, the organism being referred to herein as a subject. The subject is generally a mammal, including a human. The subject can be an individual human being in a human population. The term "mammal" as used herein includes but is not limited to a human, non-human primate, mouse, rat, dog, cat, cow, sheep, horse, and pig. Mammals other than humans can be advantageously used as subjects that can be used to provide a model of a human disease. The non-human subject can be unmodified, or a genetically modified animal (e.g., a transgenic animal, or an animal carrying one or more genetic mutation(s), or silenced gene(s)). A subject can be male or female. Depending on the objective of the operation, a subject can be one that has been exposed to an agent of interest. A subject can be one that has been exposed to an agent over an extended period of time, optionally including time prior to the study. A subject can be one that had been exposed to an agent for a period of time but is no longer in contact with the agent. A subject can be one that has been diagnosed or identified as having a disease. A subject can be one that has already undergone, or is undergoing treatment of a disease or adverse health condition. A subject can also be one that exhibits one or more symptoms or risk factors for a specific health condition or disease. A subject can be one that is predisposed to a disease, and may be either symptomatic or asymptomatic. In certain implementations, the disease or health condition in question is associated with exposure to an agent or use of an agent over an extended period of time. According to some implementations of the disclosure, a system contains or generates computerized models of one or more biological systems and mechanisms of its functions (collectively, "biological networks" or "network models") that are relevant to a type of perturbation or an outcome of interest.

Depending on the context of the operation, the biological system can be defined at different levels as it relates to the function of an individual organism in a population, an organism generally, an organ, a tissue, a cell type, an organelle, a cellular component, or a specific individual's cell(s). Each biological system comprises one or more biological mechanisms or pathways, the operation of which manifest as functional features of the system. Animal systems that reproduce defined features of a human health condition and that are suitable for exposure to an agent of interest are preferred biological systems. Cellular and organotypical systems that reflect the cell types and tissue involved in a disease etiology or pathology are also preferred biological systems. Priority could be given to primary cells or organ cultures that recapitulate as much as possible the human biology *in vivo.* It is also important to match the human cell culture *in vitro* with the most equivalent culture derived from the animal models *in vivo.* This enables creation of a translational continuum from animal model to human biology *in vivo* using the matched systems *in vitro* as reference systems. Accordingly, the biological system contemplated for use with the systems and methods described herein can be defined by, without limitation, functional features (biological functions, physiological functions, or cellular functions), organelle, cell type, tissue type, organ, development stage, or a combination of the foregoing. Examples of biological systems include, but are not limited to, the pulmonary (e.g., pulmonary inflammation), integument, skeletal, muscular, nervous (central and peripheral), endocrine, cardiovascular, immune, circulatory, respiratory, urinary, renal, gastrointestinal, colorectal, hepatic and reproductive systems. Other examples of biological systems include, but are not limited to, the various cellular functions in epithelial cells, nerve cells, blood cells, connective tissue cells, smooth muscle cells, skeletal muscle cells, fat cells, ovum cells, sperm cells, stem cells, lung cells, brain cells, cardiac cells, laryngeal cells, pharyngeal cells, esophageal cells, stomach cells, kidney cells, liver cells, breast cells, prostate cells, pancreatic cells, islet cells, testes cells, bladder cells, cervical cells, uterus cells, colon cells, and rectum cells. Some of the cells may be cells of cell lines, cultured in vitro or maintained in vitro indefinitely under appropriate culture conditions. Examples of cellular functions include, but are not limited to, cell proliferation (e.g., cell division), degeneration, regeneration, senescence, control of cellular activity by the nucleus, cell-to-cell signaling, cell differentiation, cell de- differentiation, cell stress response, autophagy, necroptosis, secretion, migration, phagocytosis, repair, apoptosis, and developmental programming. Examples of cellular components that can be considered as biological systems include, but are not limited to, the cytoplasm, cytoskeleton, membrane, ribosomes, mitochondria, nucleus, endoplasmic reticulum (ER), Golgi apparatus, lysosomes, DNA (e.g., DNA damage or DNA repair), RNA, proteins, peptides, and antibodies.

A perturbation in a biological system can be caused by one or more agents over a period of time through exposure or contact with one or more parts of the biological system. An agent can be a single substance or a mixture of substances, including a mixture in which not all constituents are identified or characterized. The chemical and physical properties of an agent or its constituents may not be fully characterized. An agent can be defined by its structure, its constituents, or a source that under certain conditions produces the agent. An example of an agent is a heterogeneous substance, that is a molecule or an entity that is not present in or derived from the biological system, and any intermediates or metabolites produced therefrom after contacting the biological system. An agent can be a carbohydrate, protein, lipid, nucleic acid, alkaloid, vitamin, metal, heavy metal, mineral, oxygen, ion, enzyme, hormone, neurotransmitter, inorganic chemical compound, organic chemical compound, environmental agent, microorganism, particle, environmental condition, environmental force, or physical force. Non-limiting examples of agents include but are not limited to nutrients, metabolic wastes, poisons, narcotics, toxins, therapeutic compounds, stimulants, relaxants, natural products, manufactured products, food substances, pathogens (prion, virus, bacteria, fungi, protozoa), particles or entities whose dimensions are in or below the micrometer range, by-products of the foregoing and mixtures of the foregoing. Non-limiting examples of a physical agent include radiation, electromagnetic waves (including sunlight), increase or decrease in temperature, shear force, fluid pressure, electrical discharge(s) or a sequence thereof, or trauma.

Some agents may not perturb a biological system unless it is present at a threshold concentration or it is in contact with the biological system for a period of time, or a combination of both. Exposure or contact of an agent resulting in a perturbation may be quantified in terms of dosage. Thus, a perturbation can result from a long-term exposure to an agent. The period of exposure can be expressed by units of time, by frequency of exposure, or by the percentage of time within the actual or estimated life span of the subject. A perturbation can also be caused by withholding an agent (as described above) from or limiting supply of an agent to one or more parts of a biological system. For example, a perturbation can be caused by a decreased supply of or a lack of nutrients, water, carbohydrates, proteins, lipids, alkaloids, vitamins, minerals, oxygen, ions, an enzyme, a hormone, a neurotransmitter, an antibody, a cytokine, light, or by restricting movement of certain parts of an organism, or by constraining or requiring exercise.

An agent may cause different perturbations depending on which part(s) of the biological system is exposed and the exposure conditions. Non-limiting examples of an agent may include a carcinogen, an irritant, an environmental pollutant, a drug, a drug candidate, or an ingredient in a consumer product, food product, beverage product, or nutritional supplement. In certain embodiments, an agent can be aerosol generated by heating tobacco, aerosol generated by combusting tobacco, tobacco smoke, cigarette smoke, any fractions thereof, and any of the gaseous constituents or particulate constituents thereof. Further non-limiting examples of an agent include compounds comprising heavy metal such as cadmium, mercury, chromium, or nicotine, tobacco-specific nitrosamines and their metabolites (4-(methylnitrosamino)-1-(3-pyridyl)-1-butanone (NNK), N'-nitrosonornicotine (NNN), N-nitrosoanatabine (NAT), N-nitrosoanabasine (NAB), 4-(methylnitrosamino)-1-(3-pyridyl)-1-butanol (NNAL)), and any product used for nicotine replacement therapy. An exposure regimen for an agent or complex stimulus should reflect the range and circumstances of exposure in everyday settings. A set of standard exposure regimens can be designed to be applied systematically to equally well-defined experimental systems. Each assay could be designed to collect time and dose-dependent data to capture both early and late events and ensure a representative dose range is covered. However, it will be understood by one of ordinary skill in the art that the systems and methods described herein may be adapted and modified as is appropriate for the application being addressed and that the systems and methods designed herein may be employed in other suitable applications.

In various implementations, high-throughput system-wide measurements for gene expression, protein expression or turnover, microRNA expression or turnover, post-translational modifications, protein modifications, translocations, antibody production metabolite profiles, or a combination of two or more of the foregoing are generated under various conditions including the respective controls. Functional outcome measurements are desirable in the methods described herein as they can generally serve as anchors for the assessment and represent clear steps in a disease etiology.

A "sample" as used herein refers to any biological sample that is isolated from a subject or an experimental system (e.g., cell, tissue, organ, or whole animal). A sample can include, without limitation, a single cell or multiple cells, cellular fraction, tissue biopsy, resected tissue, tissue extract, tissue, tissue culture extract, tissue culture medium, exhaled gases, whole blood, platelets, serum, plasma, erythrocytes, leucocytes, lymphocytes, neutrophils, macrophages, B cells or a subset thereof, T cells or a subset thereof, a subset of hematopoietic cells, endothelial cells, synovial fluid, lymphatic fluid, ascites fluid, interstitial fluid, bone marrow, cerebrospinal fluid, pleural effusions, tumor infiltrates, saliva, mucous, sputum, semen, sweat, urine, or any other bodily fluids. Samples can be obtained from a subject by means including but not limited to venipuncture, excretion, biopsy, needle aspirate, lavage, scraping, surgical resection, or other means known in the art.

**Computing Device**

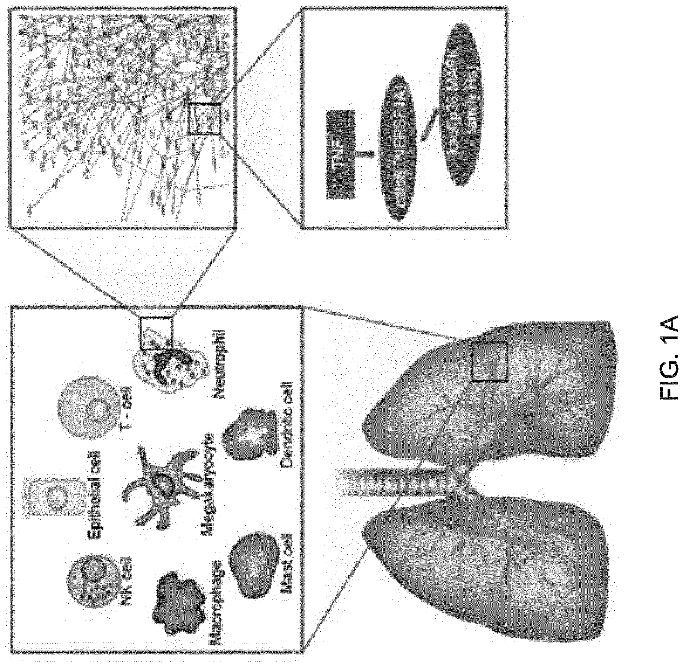

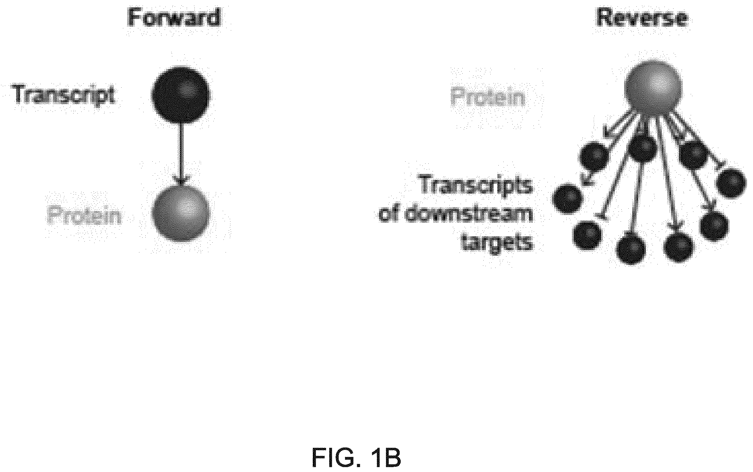

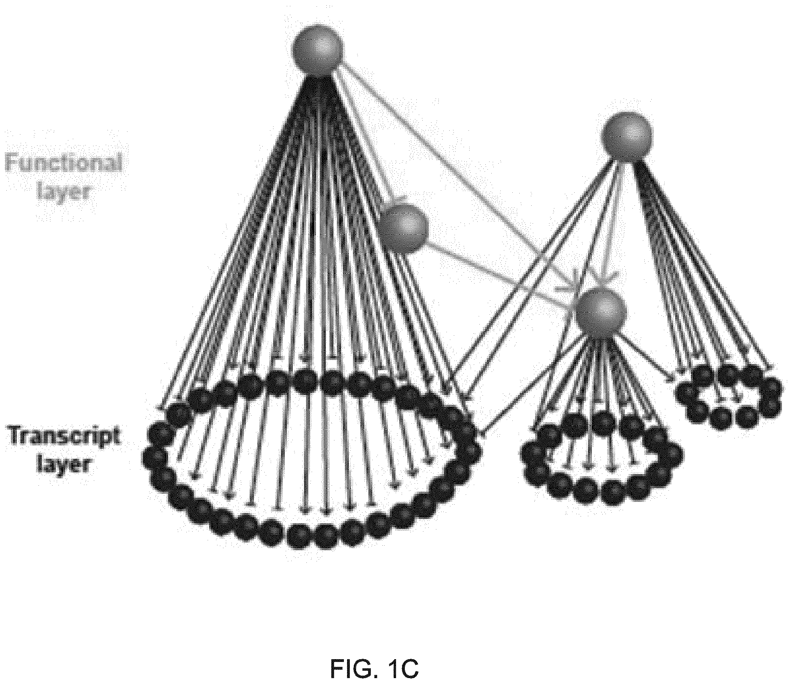

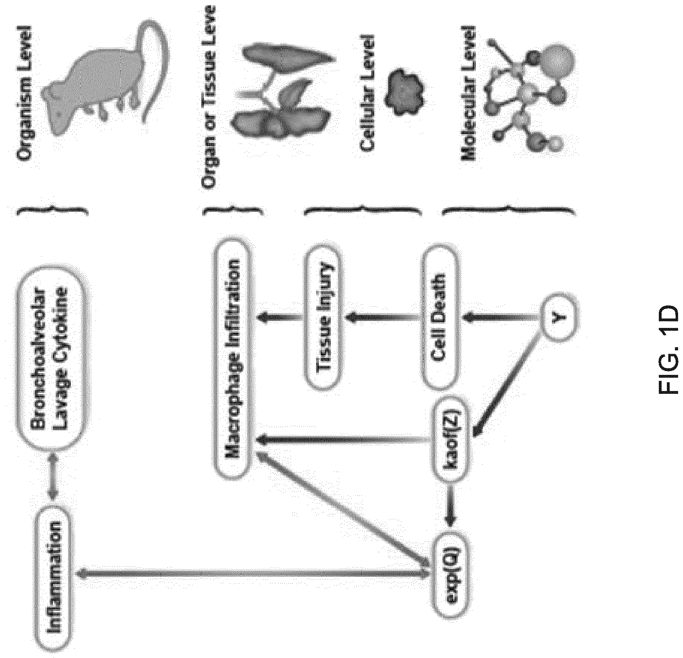

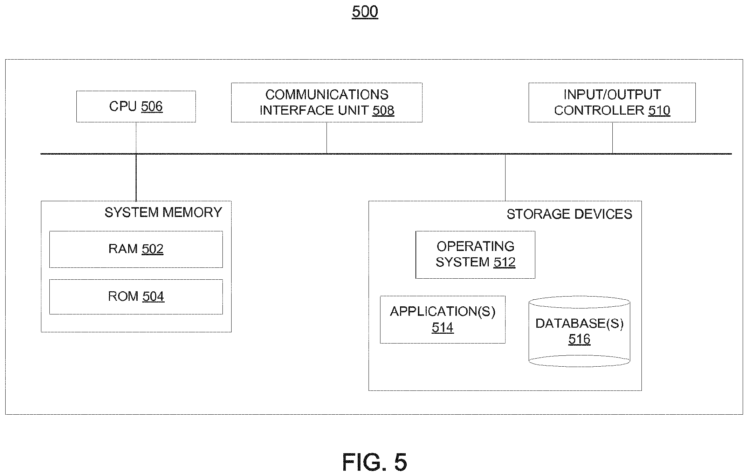

FIG. 5 is a block diagram of a computing device, such as any of the components of system 100 of FIG. 1 or system 1100 of FIG. 11 for performing processes described herein. Each of the components of system 100, including the systems response profile engine 110, the network modeling engine 112, the network scoring engine 114, the aggregation engine 116 and one or more of the databases including the outcomes database, the perturbations database, and the literature database may be implemented on one or more computing devices 500. In certain aspects, a plurality of the above-components and databases may be included within one computing device 500. In certain implementations, a component and a database may be implemented across several computing devices 500.

The computing device 500 comprises at least one communications interface unit, an input/output controller 510, system memory, and one or more data storage devices. The system memory includes at least one random access memory (RAM 502) and at least one read-only memory (ROM 504). All of these elements are in communication with a central processing unit (CPU 506) to facilitate the operation of the computing device 500. The computing device 500 may be configured in many different ways. For example, the computing device 500 may be a conventional standalone computer or alternatively, the functions of computing device 500 may be distributed across multiple computer systems and architectures. The computing device 500 may be configured to perform some or all of modeling, scoring and aggregating operations. In FIG. 5, the computing device 500 is linked, via network or local network, to other servers or systems.

The computing device 500 may be configured in a distributed architecture, wherein databases and processors are housed in separate units or locations. Some such units perform primary processing functions and contain at a minimum a general controller or a processor and a system memory. In such an aspect, each of these units is attached via the communications interface unit 508 to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to: Ethernet, SAP, SAS™, ATP, BLUETOOTH™, GSM and TCP/IP.

The CPU 506 comprises a processor, such as one or more conventional microprocessors and one or more supplementary co-processors such as math co-processors for offloading workload from the CPU 506. The CPU 506 is in communication with the communications interface unit 508 and the input/output controller 510, through which the CPU 506 communicates with other devices such as other servers, user terminals, or devices. The communications interface unit 508 and the input/output controller 510 may include multiple communication channels for simultaneous communication with, for example, other processors, servers or client terminals. Devices in communication with each other need not be continually transmitting to each other. On the contrary, such devices need only transmit to each other as necessary, may actually refrain from exchanging data most of the time, and may require several steps to be performed to establish a communication link between the devices.

The CPU 506 is also in communication with the data storage device. The data storage device may comprise an appropriate combination of magnetic, optical or semiconductor memory, and may include, for example, RAM 502, ROM 504, flash drive, an optical disc such as a compact disc or a hard disk or drive. The CPU 506 and the data storage device each may be, for example, located entirely within a single computer or other computing device; or connected to each other by a communication medium, such as a USB port, serial port cable, a coaxial cable, an Ethernet type cable, a telephone line, a radio frequency transceiver or other similar wireless or wired medium or combination of the foregoing. For example, the CPU 506 may be connected to the data storage device via the communications interface unit 508. The CPU 506 may be configured to perform one or more particular processing functions.

The data storage device may store, for example, (i) an operating system 512 for the computing device 500; (ii) one or more applications 514 (*e.g.*, computer program code or a computer program product) adapted to direct the CPU 506 in accordance with the systems and methods described here, and particularly in accordance with the processes described in detail with regard to the CPU 506; or (iii) database(s) 516 adapted to store information that may be utilized to store information required by the program. In some aspects, the database(s) includes a database storing experimental data, and published literature models.

The operating system 512 and applications 514 may be stored, for example, in a compressed, an uncompiled and an encrypted format, and may include computer program code. The instructions of the program may be read into a main memory of the processor from a computer-readable medium other than the data storage device, such as from the ROM 504 or from the RAM 502. While execution of sequences of instructions in the program causes the CPU 506 to perform the process steps described herein, hard-wired circuitry may be used in place of, or in combination with, software instructions for implementation of the processes of the present disclosure. Thus, the systems and methods described are not limited to any specific combination of hardware and software.

Suitable computer program code may be provided for performing one or more functions in relation to modeling, scoring and aggregating as described herein. The program also may include program elements such as an operating system 512, a database management system and "device drivers" that allow the processor to interface with computer peripheral devices (*e.g.*, a video display, a keyboard, a computer mouse, etc.) via the input/output controller 510.

The term "computer-readable medium" as used herein refers to any non-transitory medium that provides or participates in providing instructions to the processor of the computing device 500 (or any other processor of a device described herein) for execution. Such a medium may take many forms, including but not limited to, non-volatile media and volatile media. Non-volatile media include, for example, optical, magnetic, or opto-magnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU 506 (or any other processor of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device 500 (*e.g.*, a server) can receive the data on the respective communications line and place the data on a system bus for the processor. The system bus carries the data to main memory, from which the processor retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

## Mechanistic Network Models

Using computational network models to interpret omics data, such as transcriptomic data, provides a more detailed molecular understanding of biological network perturbations by extracting mechanistic information from systems biology datasets. Omics data refers to biological data obtained typically by techniques that allow measurements to be made, often simultaneously, at a system-wide scale covering a substantial number of members of a class of biological molecules. Examples of omics data that can be used in the present invention include but are not limited to those obtained by techniques applied within the study of genomics, epigenomics, proteomics, transcriptomics, lipidomics and metabolomics. A three- step model building process can be used. In particular, the network models consist of qualitative causal relationships to present biological processes. FIG. 1A illustrates one such network model describing tissue and cell-specific biological processes in the form of causal relationships between biological entities. The models are coded in Biological Expression Language (BEL) and in a database, which represents scientific findings in a computable format. The BEL framework is an open-source technology for managing, publishing, and using structured life-science knowledge, but in general, any suitable framework may be used. The BEL framework is unlike the BioPAX (Biological Pathway Exchange) that focuses on the integration and exchange of biological pathway data across a large array of existing pathway resources [Demir, E. et al. (2010) The BioPAX community standard for pathway data sharing. Nature Biotechnology. 28, 935-942]. Pathway-based approaches , such as KEGG (Kyoto Encyclopedia of Genes and Genomes) [Kanehisa, M. et al. (2012) KEGG for integration and interpretation of large-scale molecular data sets. Nucleic Acids Res. 40, D109-14] and IPA (Ingenuity Pathway Analysis) (www.ingenuity.com), identify differentially expressed genes whose protein products act in a pathway of interest.

A biological system can be modeled as a mathematical graph consisting of vertices (or nodes) and edges that connect the nodes. The nodes can represent biological entities within a biological system, such as, but not limited to, compounds, DNA, RNA, proteins, peptides, antibodies, cells, tissues, and organs. The edges can represent relationships between the nodes. The edges in the graph can represent various relations between the nodes. For example, edges may represent a "binds to" relation, an "is expressed in" relation, an "are co-regulated based on expression profiling" relation, an "inhibits" relation, a "co-occur in a manuscript" relation, or "share structural element" relation. Generally, these types of relationships describe a relationship between a pair of nodes. The nodes in the graph can also represent relationships between nodes. Thus, it is possible to represent relationships between relationships, or relationships between a relationship and another type of biological entity represented in the graph. For example a relationship between two nodes that represent chemicals may represent a reaction. This reaction may be a node in a relationship between the reaction and a chemical that inhibits the reaction.

A graph may be undirected, meaning that there is no distinction between the two vertices associated with each edge. Alternatively, the edges of a graph may be directed from one vertex to another. For example, in a biological context, transcriptional regulatory networks and metabolic networks may be modeled as a directed graph. In a graph model of a transcriptional regulatory network, nodes would represent genes with edges denoting the transcriptional relationships between them. As another example, protein-protein interaction networks describe direct physical interactions between the proteins in an organism's proteome and there is often no direction associated with the interactions in such networks. Thus, these networks may be modeled as undirected graphs. Certain networks may have both directed and undirected edges. The entities and relationships (*i.e.*, the nodes and edges) that make up a graph, may be stored as a web of interrelated nodes in a database.

The knowledge represented within the database may be of various different types, drawn from various different sources. For example, certain data may represent a genomic database, including information on genes, and relations between them. In such an example, a node may represent an oncogene, while another node connected to the oncogene node may represent a gene that inhibits the oncogene. The data may represent proteins, and relations between them, diseases and their interrelations, and various disease states. There are many different types of data that can be combined in a graphical representation. The computational models may represent a web of relations between nodes representing knowledge in, *e.g.*, a DNA dataset, an RNA dataset, a protein dataset, an antibody dataset, a cell dataset, a tissue dataset, an organ dataset, a medical dataset, an epidemiology dataset, a chemistry dataset, a toxicology dataset, a patient dataset, and a population dataset. As used herein, a dataset is a collection of numerical values resulting from evaluation of a sample (or a group of samples) under defined conditions. Datasets can be obtained, for example, by experimentally measuring quantifiable entities of the sample; or alternatively, or from a service provider such as a laboratory, a clinical research organization, or from a public or proprietary database. Datasets may contain data and biological entities represented by nodes, and the nodes in each of the datasets may be related to other nodes in the same dataset, or in other datasets. Moreover, the network modeling engine may generate computational models that represent genetic information, in, *e.g.*, DNA, RNA, protein or antibody dataset, to medical information, in medical dataset, to information on individual patients in patient dataset, and on entire populations, in epidemiology dataset. In addition to the various datasets described above, there may be many other datasets, or types of biological information that may be included when generating a computation model. For example, a database could further include medical record data, structure/activity relationship data, information on infectious pathology, information on clinical trials, exposure pattern data, data relating to the history of use of a product, and any other type of life science-related information.

The changes in gene expression do not always correlate with changes in protein activity. The network models as described herein do not necessarily rely on these "forward assumptions", but rather may infer the activity of an upstream node based on the expression of genes that the node regulates. FIG. 1B illustrates a causal biological network model comprising backbone nodes and supporting nodes for indicating cause and effect relationships. "Forward reasoning" assumes that gene expression correlates with changes in protein activity, whereas "backward reasoning" or "reverse causal reasoning" considers the changes in gene expression as the consequence of the activity of an upstream entity. In various implementations, the activity of the nodes within such network models may be predicted based on an underlying measurable layer, which are the differentially expressed genes, whose function does not need to be known. FIG. 1C illustrates a network model that captures biology in the nodes and causal relationships between the nodes. Differential expressions of genes (small black balls) are experimental evidence for the activation of an upstream node.

The network models used in the present invention comprising nodes that indicate cause and effect based on reverse causal reasoning contains several advantages. First, nodes in the network are connected by causally related edges with fixed topology, allowing the biological intent of the network model to be easily digested by a scientist or a user, enabling inference and computation on the network as a whole. Second, unlike other approaches for building pathway or connectivity maps where connections are often represented out of a tissue or disease context, the network models herein obey appropriate tissue/cell context and biological processes. Third, the causal network models can capture changes in a wide range of biological molecules including proteins, DNA variants, coding and non-coding RNA, and other entities, such as phenotypic, chemicals, lipids, methylation states or other modifications (e.g., phosphorylation), as well as clinical and physiological observations. FIG. 1D illustrates a network model representative of knowledge from molecular, cellular, and organ levels to an entire organism. Fourth, the network models are evolving and can be modified to represent specific species and/or tissue contexts by the application of appropriate boundaries and updated as additional knowledge becomes available. Fifth, the network models are transparent; the edges (cause and effect relationships) in the network model are all supported by published scientific findings anchoring each network to the scientific literature for the biological process being modeled. Finally, the network models can be provided in (.XGMML) format to allow easy visualization using freely available tools including Cytoscape [Smoot, M.E. et al. (2011) Cytoscape 2.8: new features for data integration and network visualization. Bioinformatics. 27, 431-432].

The network model is used as a substrate for simulation and analysis, and is representative of the biological mechanisms and pathways that enable a feature of interest in the biological system. The feature or some of its mechanisms and pathways may contribute to the pathology of diseases and adverse effects of the biological system. Prior knowledge of the biological system represented in a database is used to construct the network model which is populated by data on the status of numerous biological entities under various conditions including under normal conditions and under perturbation by an agent. The network model used is dynamic in that it represents changes in status of various biological entities in response to a perturbation and can yield quantitative and objective assessments of the impact of an agent on the biological system.

**Assessment of a Network Perturbation Amplitude (NPA) score**

Certain implementations of the present disclosure include methods for computing a numerical value that expresses the magnitude of changes within a portion of a biological system. The computation uses as input, a set of data obtained from a set of controlled experiments in which the biological system is perturbed by an agent. The data is then applied to a network model of a feature of the biological system.

The numerical values generated by computerized methods of the disclosure can be used to determine the magnitude of desirable or adverse biological effects caused by manufactured products (for safety assessment or comparisons), therapeutic compounds including nutrition supplements (for determination of efficacy or health benefits), and environmentally active substances (for prediction of risks of long term exposure and the relationship to adverse effect and onset of disease), among others.

In one aspect, the systems and methods described herein provide a computed numerical value representative of the magnitude of change in a perturbed biological system based on a network model of a perturbed biological mechanism. The numerical value referred to herein as a network perturbation amplitude (NPA) score can be used to summarily represent the status changes of various entities in a defined biological mechanism. NPA has been previously described in detail in U.S. Provisional Patent Application No. 61/525,700 (Attorney docket number FTR0689 / 106500-0011-001), 61/527,946 (Attorney docket number FTR0751 / 106500-0015-001), and 61/532,972 (Attorney docket number FTR0748 / 106500-0016-001) and PCT Application No. PCT/EP2012/061035 (Attorney docket number FTR0689 / 106500-0011-WO1), PCT/EP2012/066557 (Attorney docket number FTR0751 / 106500-0015-WO1), and PCT/EP2012/003760 (Attorney docket number FTR0748 / 106500-0016-WO1). The numerical values obtained for different agents or different types of perturbations can be used to compare relatively the impact of the different agents or perturbations on a biological mechanism which enables or manifests itself as a feature of a biological system. Thus, NPA scores may be used to measure the responses of a biological mechanism to different perturbations.

The NPA scores may assist researchers and clinicians in improving diagnosis, experimental design, therapeutic decision, and risk assessment. For example, the NPA scores may be used to screen a set of candidate biological mechanisms in a toxicology analysis to identify those most likely to be affected by exposure to a potentially harmful agent. By providing a measure of network response to a perturbation, these NPA scores may allow correlation of molecular events (as measured by experimental data) with phenotypes or biological outcomes that occur at the cell, tissue, organ or organism level. A clinician may use NPA values to compare the biological mechanisms affected by an agent to a patient's physiological condition to determine what health risks or benefits the patient is most likely to experience when exposed to the agent (e.g., a patient who is immuno-compromised may be especially vulnerable to agents that cause a strong immuno-suppressive response).

According to an illustrative implementation of the disclosure, the causal network models are combined with algorithms for computing NPA scores. As a result, gene expression fold-changes, also referred to as a set of contrasts or contrast data, are translated into differential values for each node of a network (denoted by *f*). The node differential values are in turn summarized into a quantitative measure of NPA.

The NPA can be computed as a Sobolev-type (semi-) norm on the signed directed graph underlying the network (*N*), which can be expressed as a quadratic form /#edges $f^T Q_N f$. That is, if the vector of activity values associated with the set of backbone entities is denoted $f_2$, the NPA score can be calculated via the quadratic form

$$NPA = f_2^T Q f_2$$

where

$$Q = \left(diag\left(out|_{l^2(v \backslash v_0)}\right) + diag\left(in|_{l^2(v \backslash v_0)}\right) - \left(-A - A^T\right)\right)|_{l^2(v \backslash v_0)} \in l^2(V \backslash V_o)$$

*diag(out)* denotes the diagonal matrix with the out-degree of each node in the second set of nodes, *diag(in)* denotes the diagonal matrix with the in-degree of each node in the second set of nodes, *V* is the set of all nodes in the network, and *A* denotes the adjacency matrix of the computational network model limited to only nodes representing backbone entities and defined in accordance with

$$A_{xy} = \begin{cases} sign(x \to y) \text{ if } x \to y \\ \quad\quad 0 \quad\quad \text{else} \end{cases}$$

If *A* is a weighted adjacency matrix, then element *(x,y)* of *A* may be multiplied by a weight factor *w(x→y)*. In some scenarios, some backbone nodes may have more supporting gene expression evidence than other backbone nodes due to the so-called literature bias in which some entities are studied more than others. The result in the causal computation biological model is that nodes with more supporting evidence will have a higher degree then less "rich" nodes. When compounded with the possibility that a majority of the evidence have very low signal, the inferred node activity values might be systematically one of the nodes with the lowest value. To address this issue, in some implementations, the weights associated with an edge from a node to one of the node's *N* downstream nodes is set to *1/N*. This modification may advantageously emphasize the backbone structure (which captures important aspects of the biology) and balance the importance of the backbone and the supporting nodes within the causal biological network model computations.

In some implementations, the NPA score is calculated in accordance with

$$NPA(G, \beta) = \frac{1}{|\{x \to y\} \text{ s. t. } x, y \notin V_0|} \sum_{\substack{x \to y \\ \text{s.t. } x,y \notin V_0}} (f(x) + sign(x \to y)f(y))^2$$

where $V_o$ denotes the set of supporting entities (*i.e.*, those for which treatment and control data are received), *f(x)* denotes the activity value for the biological entity *x*, and *sign(x→y)* denotes the direction value of the edge in the computational network model that connects the node representing biological entity *x* to the node representing biological entity *y*.

Thus, the NPA algorithm considers two main input components, whereby one is the causal network model describing the mechanism and the other a gene expression data set from a well-designed experiment. The backbone scores for a given contrast and a given network $N_i = (V_i, E_i, s_i)$ (nodes, edges, edge signs), denoted by $f_i$, lies in $l^2(Vi)$ endowed with the scalar product $<.|.>_i$ given by $Q_i$.

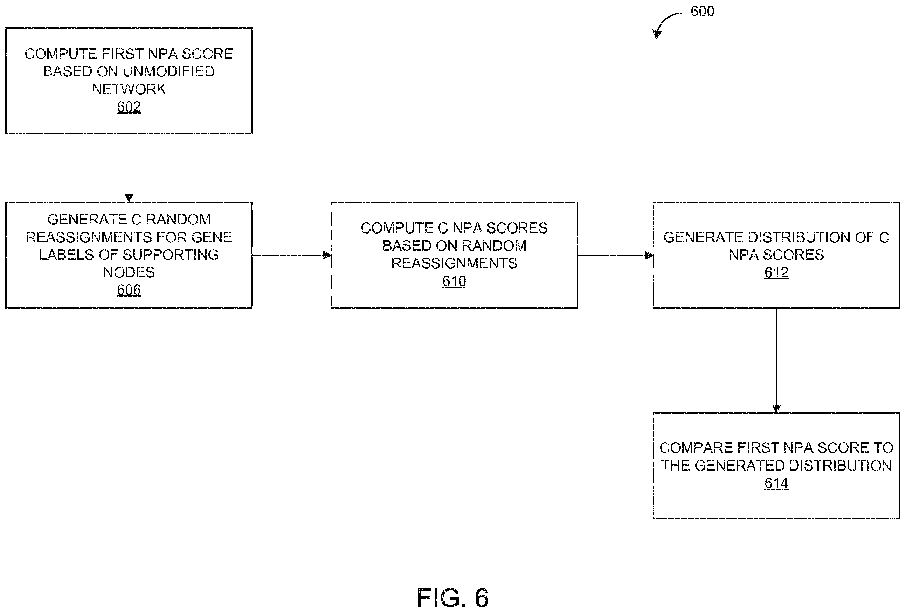

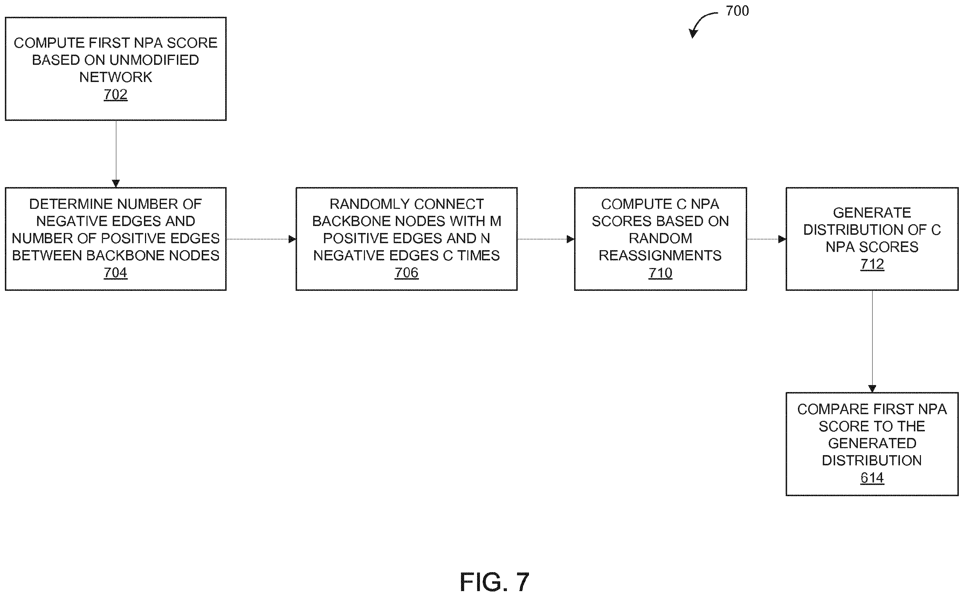

<u>Determining a statistical significance of an NPA score</u> In addition to the confidence intervals of the NPA scores, which can account for experimental error (e.g., biological variation between samples in an experimental group), companion statistics can be derived to inform the specificity of the NPA score with respect to the biology described in the network. In particular, two permutation tests may be implemented. Example processes for performing the two permutations tests are described in detail in relation to FIGS. 6 and 7. One purpose of each permutation test is to determine a statistical significance of a proposed NPA score, which can be usefulf or indicating whether the biological system that is being modeled by the network has been perturbed. Both tests involve generating random permutations of one or more aspects of the causal network model, using the resulting test models to compute test NPA scores based on the same datasets and algorithms that generated the proposed NPA score, and comparing or ranking the test NPA scores with the proposed NPA score to determine statistical significance of the proposed NPA score. The aspects of a causal network model that may be randomly assorted to generate the test models include the labels of the supporting nodes, the edges connecting the backbone nodes to the supporting nodes, or the edges that connect backbone nodes to each other. To determine the statistical significance of a proposed NPA score, the network scoring engine may subject the data to one or both tests as described below.

The first test assesses whether the results are specific to the underlying evidence (i.e., gene fold-changes) in the model, leading to a permutation *P*-value (denoted by *O in the figures when <0.05). The first test may be referred to as an "O" statistic analysis and assesses the importance of the positions of the supporting nodes within the causal network model. The process 600 described in relation to FIG. 6 is an example method for computing an "O"-statistic, according to an illustrative implementation of the disclosure.

The "O-statistic" test, assesses the importance of the positions of the supporting nodes within the causal network model. The process 600 includes a method to assess the statistical significance of a computed NPA score. In particular, at step 602, a first proposed NPA score is computed based on the network based on knowledge of causal relationship of entities in the biological system, also referred to as an unmodified network. At step 606, the gene labels and as a result the corresponding values of each supporting node are randomly reassigned among the supporting nodes in the network model. The random reassignment is repeated a number of times, e.g., C times, and at step 612, the test NPA scores are computed based on the random reassignments, resulting in a distribution of C test NPA scores. The network scoring engine may compute the proposed and test NPA scores according to any of the methods described above for computing an NPA score based on the network. At step 614, the proposed NPA score is compared to or ranked against the distribution of test NPA scores to determine the statistical significance of the proposed NPA score.

The methods of quantifying the perturbation of a biological system comprise computing a proposed NPA score based on a causal network model, and determining the statistical significance of the score. The significance can be computed by a method comprising reassigning randomly the labels of the supporting nodes of a causal network model to create a test model, computing a test NPA score based on a test model, and comparing the proposed NPA score and the test NPA scores to determine whether the biological system is perturbed. The labels of the supporting nodes are associated with the activity measures.

The integer C may be any number determined by the network scoring engine and may be based on a user input. The integer C may be sufficiently large such that the resulting distribution of NPA scores based on the random reassignments is approximately smooth. The integer C may be fixed such that the reassignments are performed a predetermined number of times. Alternatively, the integer C may vary depending on the resulting NPA scores. For example, the integer C may be iteratively increased, and additional reassignments may be performed if the resulting NPA distribution is not smooth. In addition, any other additional requirements for the distribution may be used, such as increasing C until the distribution resembles a certain form, such as Gaussian or any other suitable distribution. In certain implementations, the integer C ranges from about 500 to about 1000.

At step 610, the network scoring engine computes C NPA scores based on the random reassignments generated at step 606. In particular, an NPA score is computed for each reassignment generated at step 606. In certain implementations, all the C reassignments are first generated at step 606, and then the corresponding NPA scores are computed based on the C reassignments at step 610. In other implementations, a corresponding NPA score is computed after each set of reassignment is generated, and this process is repeated C times. The latter scenario may save on memory costs and may be desirable if the value for C is dependent on previously computed N values. At step 612, the network scoring engine aggregates the resulting C NPA scores to form or generate a distribution of NPA values, corresponding to the random reassignments generated at step 606. The distribution may correspond to a histogram of the NPA values or a normalized version of the histogram.

At step 614, the network scoring engine compares the first NPA score to the distribution of NPA scores generated at step 612. As an example, the comparison may include determining a "p-value" representative of a relationship between the proposed NPA score and the distribution. In particular, the p-value may correspond to a percentage of the distribution that is above or below the proposed NPA score value. A p-value that is small, for example less than 0.5%, less than 1%, less than 5%, or any other fraction, indicates that the proposed NPA score is statistically significant. For example, a proposed NPA score with a low p-value (<0.05 or below 5%, for example) computed at step 1 indicates that the proposed NPA score is high relative to a significant number of the test NPA scores resulting from the random gene label reassignments.

The second test assesses whether the "cause-and-effect" layer of the network significantly contributes to the amplitude of network perturbation (denoted by K* in the figures when <0.05). The network is considered to be specifically perturbed if both $P$-values are low (typically <0.05), and the perturbation is called significant if, *in addition,* the confidence interval is above zero. The second test may be referred to as a "K" statistic analysis and assesses the importance of the structure of the backbone nodes within the causal network model. The process 700 described in relation to FIG. 7 is an example method for computing a "K"-statistic, according to an illustrative implementation of the disclosure.

The "K-statistic" test, assesses the importance of the structure of the backbone nodes within the causal network model. The process 700 includes a method to assess the statistical significance of a proposed NPA score. The process 700 is similar to the process 600 in that an aspect of the causal network model is randomly assorted to create a plurality of test models whereupon a plurality of test NPA scores are computed. The causal network model that is built on knowledge of causal relationship of entities in the biological system, also referred to as an unmodified network. In such a model, an edge may be signed, and thus an edge may represent a positive or negative relationship between two backbone nodes. Accordingly, the causal network model comprises n edges that connect backbone nodes resulting in a positive influence, and m edges that connect backbone nodes resulting in a negative influence.

At step 702, a proposed NPA score is computed based on the network built on knowledge of causal relationship of entities in the biological system. Then, at step 704, a number n of negative edges and a number m of positive edges are determined. At step 706, pairs of backbone nodes are each randomly connected with one of the n negative edges or one of the m positive edges. This process of generating the random connections with n + m number of edges is repeated C times. As previously described, the number of iterations C, can be determined by user input or by the smoothness of the distribution of test NPA scores. At step 712, a plurality of test NPA scores are computed based on a plurality of test models comprising backbone nodes that are connected randomly to other backbone nodes. The network scoring engine may compute the proposed and test NPA scores according to any of the methods described above for computing an NPA score based on the network. At step 714, the proposed NPA score is compared to or ranked against a distribution of test NPA scores to determine the statistical significance of the proposed NPA score.

At step 710, the network scoring engine computes C NPA scores based on the random reconnections formed at step 706. At step 712, the network scoring engine aggregates the resulting C NPA scores to generate a distribution of test NPA values, based on the test models resulting from the random reconnections generated at step 606. The distribution may correspond to a histogram of the NPA values or a normalized version of the histogram.

At step 714, the network scoring engine compares the proposed NPA score to the distribution of NPA scores generated at step 712. As an example, the comparison may include determining a "p-value" representative of a relationship between the proposed NPA score and the distribution. In particular, the p-value may correspond to a percentage of the distribution that is above or below the proposed NPA score value. A p-value that is small, for example less than 0.1%, less than 0.5%, less than 1%, less than 5%, or any intermediate fractions, indicates that the proposed NPA score is statistically significant. For example, a proposed NPA score with a low p-value (<0.05 or below 5%, for example) computed at step 714 indicates that the proposed NPA score is high relative to a significant number of the test NPA scores resulting from the random reconnections of backbone nodes.

In certain implementations, it may be required that both p-values (computed in FIGS. 6 and 7) are low for the proposed NPA score to be considered statistically significant. In other implementations, the network scoring engine may require one or more p-values to be low in order to find the proposed NPA score to be significant.

## Assessment of a Biological Impact Factor (BIF) score

According to an illustrative implementation of the disclosure, a processor generates a "biological impact factor" or "BIF" based on one or more NPA scores, each representative of a perturbation of a biological system based on datasets and a plurality of computational models. The computerized methods described herein aggregate a plurality of network response scores, taking into account the relative contribution of each network to the overall status of the biological system, to produce a numerical value, i.e., a BIF given a plurality of datasets. In various implementations, the computerized method of the present disclosure combines a plurality of model scores corresponding to the plurality of treatments (or agents), adjusts the scores according to the intersection effects of overlapping network, and generates a BIF that represents the relative biological effects caused by the treatments (or agents). One aspect of BIF which does not take into account the intersection effect of overlapping networks has been previously described in detail in U.S. Provisional Patent Application No. 61/495,824 (Attorney docket number FTR0690 / 106500-0010-001) and PCT Application No. PCT/EP2012/061033 (Attorney docket number FTR0690 / 106500-0010-WO1).

The BIFs generated by the computerized methods disclosed herein can be used to estimate or determine the magnitude of desirable or adverse biological effects caused by any external factors, including but not limited to pathogens (for diagnosis or prognosis of diseases), harmful substances (for toxicological assessment), manufactured products (for safety assessment or risk-of-use comparisons), therapeutic compounds including nutrition supplements (for determination of efficacy or health benefits), and changes in the environment or environmentally active substances (for public health assessment, e.g., pollutants or ultraviolet light from the sun). The BIF can be used for prediction of the biological risks of acute, intermittent or sustained exposure, and the relationship to immediate or long term adverse effects on the biological system and onset of disease(s). The perturbation is a cause that is external to the biological system in question.

BIF values obtained for different agents or different types of perturbations can be used to compare relatively the impact of the different agents or perturbations on a biological system. A BIF can be used as a predictor for medium and long term disease outcome, optionally the value can be calibrated using a combination of experimental and epidemiological data. BIF values are computed by using any of various mathematical and computational algorithms known in the art according to the methods disclosed herein, employing one or more datasets obtained from one or more sample or subject.

A BIF value can be computed which represent the differential response of a biological system to at least two different treatment conditions. In some embodiments, a first treatment condition can be a perturbation regarded as an experimental treatment (such as but not limited to exposure to a potentially carcinogenic agent) and a second treatment condition regarded as a control (such as a null treatment). In some embodiments, the BIF computed to represent the impact of a first agent in a biological system can be used for comparison with the BIF representing the impact of a second agent in the same biological system. The numerical scores can thus be used to assess and compare the differential effects of two or more agents on a biological system or certain features thereof. Accordingly, a plurality of datasets is obtained from measurements of changes in the biological system after it has been exposed respectively to a plurality of different agents and/or under different environmental conditions.

The network models represent functionally distinct biological processes that characterize features of the biological systems under consideration. To objectively evaluate the overall biological impact relative to a reference within the experiment (for example, a standardized cigarette smoke exposure), all the backbone scores, $f_1,..,f_m$, for networks $N_1,...,N_m$ are considered. The Hilbert space, $\mathcal{H}$, under consideration will be the direct-sum (not necessarily orthogonal direct sum) of the $(l^2(Vi), <.|.>_i) : \oplus_i l^2(Vi)$. A new scalar product may then be defined on this direct sum. Let $w_i$ be a positive weight associated with network $N_i$. In some implementations, $w_i$ takes a binary value, depending on the "O" and "K" statistics described in relation to FIGS. 6 and 7: $w_i=1$ if both p-values are <0.05 and 0 else. In particular, one can consider the orthogonal direct sum for which the new scalar product will be defined by $<f|g>_{\mathcal{H}} = \sum_i w_i < f_i|g_i>_{l2(Vi)}$. More generally, the scalar product on $\mathcal{H}= \mathcal{H}(\{N_1,\ldots,N_m\})$ will be non-orthogonal and denoted by $<.|.>_{\mathcal{H}}$

The scalar product will account for overlap between the networks and can be used to adjust the scores in view of an effect of the intersection of overlapping networks. Some nodes and/or edges are shared between networks and hence introducing a non-orthogonal direct sum will allow accounting for this fact. One possible choice is given hereafter: Let $\theta_1^j:l^2(V_i) \to l^2 (V_i \cap V_j)$, defined by restriction. Scalar product is defined by restriction as well. Then $\|f\|^2_{\mathcal{H}}$ can be defined by $\sum_i w_i \|f_i\|^2_{l2(Vi)} - 1/m \sum_{i,j;i\neq j} w_i w_j < \theta_i^j(f_i)| \theta_j^i(f_j)>$. This corresponds to the scalar product: $\sum_i w_i < f_i|g_i >_{l2(Vi)} - 1/m \sum_{i,j;i\neq j} w_i w_j < \theta_i^j(f_i)| \theta_j^i(g_j)>$. It can be shown that this bilinear form is positive definite and hence defines a scalar product. The rationale for this choice is to cancel out the intersection effect between

$N_i$ and $N_j$ when the backbone scores match on the intersection, while keeping each contribution if those are orthogonal

$$< \theta_i^j(f_i) \mid \theta_j^i(f_j)> = 0$$

. Any another suitable scalar product may also be defined by a block matrix whose block-diagonal is given by the $Q_i$'s and extra diagonal block to reflect the intersections.

Consider the partition of all the network set $(N_1,...,N_M)$ into subset of networks, $S_1,..S_b$; $b \leq M$. A subset of networks may characterize a particular biological process such as inflammation, cell stress, senescence, or any other suitable biological process or feature of a biological system.

For a given contrast, c, (treatment vs. control), the impact $BIF(c,S_k)$ of the subset $S_k$ is computed as the square-norm of the backbone scores in the corresponding direct sum (as defined above). By considering the orthogonal direct sum over the subset S of the non-orthogonal sums within the subsets, an overall Hilbert space, $\mathcal{H}_{BIF}$, is defined, accounting for all the networks and their subsets; for which a scalar product is defined as: $\mathcal{H}_{BIF} = \bigoplus_k \mathcal{H}(S_k)$.

Let REF denote a contrast of reference (typically a standardized CS exposure). Then the relative BIF, RBIF, is defined as the ratio of the norm of the vector corresponding to c and the reference vector, REF. It can be computed as:

$$RBIF(c) = \frac{\|f_c\|_{\mathcal{H}_{BIF}}^2}{\|f_{REF}\|_{\mathcal{H}_{BIF}}^2} = \frac{\sum_k BIF(c, S_k)}{\sum_k BIF(REF, S_k)}$$

The contribution of the subset S is defined by:

$$Contrib_s(c) = \frac{BIF(c, S)}{\sum_k BIF(c, S_k)}$$

These contributions sum to 1.

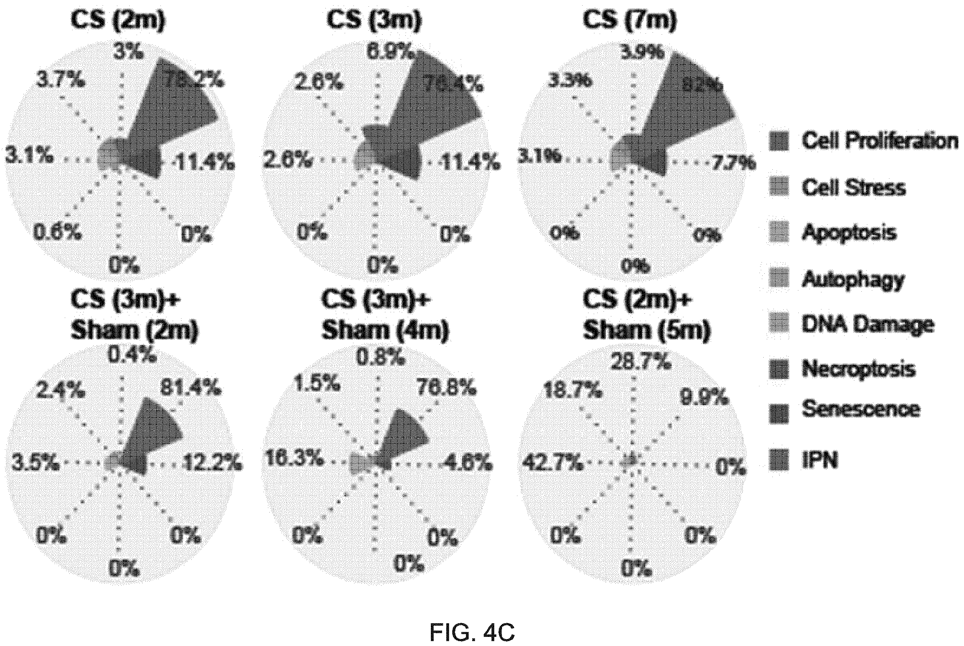

The relative BIF is therefore decomposed into network components (namely cell stress, pulmonary inflammation, cell proliferation, apoptosis, necroptosis, senescence, DNA damage, and autophagy, but any other suitable network component may also be used in addition to or in place or any of the named network components) by considering the quantities *Contribs(c) RBIF(c)*, which can be represented as starplots as shown in FIG. 4C.

Finally, because RBIF is an aggregated quantity, the two contrasts, c and REF, can have the same relative biological effect while arising from different network models. To identify those situations, a coefficient δ may be computed as:

$$\delta = \frac{< f_c \mid f_{REF} >_{\mathcal{H}_{BIF}}}{\|f_c\|_{\mathcal{H}_{BIF}} \cdot \|f_{REF}\|_{\mathcal{H}_{BIF}}}$$

This coefficient is the cos angle between the contrast c and the reference contrast REF for the scalar product defined in $\mathcal{H}_{BIF}$.

According to an illustrative implementation, a BIF may be computed as follows for an orthogonal direct sum case. The sum of the significant network perturbations for the contrast c is normalized with respect to the corresponding sum for the reference. Hence, the relative BIF (RBIF) for the contrast c is defined as:

$$RBIF(i) = \frac{\sum_{Net} w_C^{Net} f_C^{Net^T} Q_{Net} f_C^{Net}}{\sum_{Net} w_{REF}^{Net} f_{REF}^{Net^T} Q_{Net} f_{REF}^{Net}} = \frac{\sum_{Net} w_C^{Net} NPA_{Net}(c)}{\sum_{Net} w_{REF}^{Net} NPA_{Net}(REF)}$$

where the weights account for three statistics associated with the above outlined NPA algorithm.

The contribution of a given subset, S, of network models (e.g., cell stress subnetworks), for a contrast *c*, is:

$$Contrib_s(c) = \frac{\sum_S w_i^S NPA_S(c)}{\sum_{Net} w_c^{Net} NPA_{Net}(c)}$$

(because Nets is a disjoint union of subsets of networks, the contributions sum to one).

The comparability coefficient becomes:

$$\delta = \frac{\sum_{Net} w_c^{Net} w_{REF}^{Net} f_c^{Net^T} Q_{Net} f_{REF}^{Net}}{\sqrt{\sum_{Net} w_c^{Net} NPA_{Net}(c)} \sqrt{\sum_{Net} w_{REF}^{Net} NPA_{Net}(REF)}}$$

**Materials and methods**

In accordance with an implementation of the disclosure, C57BL6 mice (8 female per group) were exposed to mainstream smoke from the Kentucky reference cigarette, 3R4F (750 $\mu$g TPM/l, 4 h/day with fresh air breaks, 5 days a week), or to fresh air (sham) for up to 7 months. Following 2- and 3-month exposure to 3R4F, subgroups of mice were exposed to fresh air for a 3-, 4-, or 5-month cessation period.

For each lung specimen, 22 slices (each 20 $\mu$m) were cut with a cryostat and homogenized. Total RNA was extracted, quality checked, and hybridized to an Affymetrix MG430 2.0 chip. After array scanning, hybridization intensity data were extracted and stored in cel files. An initial chip quality check was also performed using the Affymetrix software.

Bronchoalveolar lavage fluid (BALF) was collected. Briefly, the lungs from 8 mice were lavaged 5 times. The supernatant was aliquoted and multianalyte profiling was performed by Myriad RBM (Myriad RBM; Austin, TX, USA) using a multiplexed bead array according to the RodentMAP v. 2.0 program. For the second to the fifth lavage cycles, 1 ml of PBS with 0.325% BSA was used for each cycle. In addition, the cell pellets of the 5 cycles were pooled, counted and differentiated using a FACScanto flow cytometer (BD Biosciences, San Jose, CA, USA) for free lung cell count (FLC)/viability.

Data processing and scoring methods were implemented in the R statistical environment [R Development Core Team, R: A Language and Environment for Statistical Computing. 2009]. Raw RNA expression data were analyzed using the *affy* and *limma* packages of the Bioconductor suite of microarray analysis tools available in the R statistical environment [Gentleman, R., Bioinformatics and computational biology solutions using Rand Bioconductor. 2005, New York: Springer Science+Business Media. xix, 473 p; Gentleman, R.C. et al. (2004) Bioconductor: open software development for computational biology and bioinformatics. Genome Biol. 5, R80]. Robust Multichip Average (GCRMA) background correction and quantile normalization were used to generate probe set expression values [Irizarry, R.A. et al. (2003) Exploration, normalization, and summaries of high density oligonucleotide array probe level data. Biostatistics. 4, 249-64]. For each data set, an overall linear model was fit to the data for all groups of replicates, and specific contrasts of interest (comparisons of "treated" and "control" conditions) were evaluated to generate raw *p*-values for each probe set on the expression array, which in turn are adjusted by the Benjamini-Hochberg procedure. These methods were applied to two test cases which are referred to below as case 1 and case 2.

**Case 1: The xenobiotic metabolism network as a biomarker for CS exposure across *in vivo* and *in vitro* systems**

Humans and other mammals are equipped with sophisticated machinery to address carcinogens and other toxic compounds; the defense system consists of oxidative reactions by cytochrome P450 enzymes (CYP; phase 1) followed by conjugation reactions by phase 2 enzymes. These enzymes sequentially convert lipophilic chemical compounds into a hydrophilic, water-soluble form that can be eliminated from the body [Burchell, B. et al. (2005) Substrate Specificity of Human Hepatic Udp-Glucuronosyltransferases. Methods inenzymology. 400, 46-57; Guengerich, F.P. (2001) Common and uncommon cytochrome P450reactions related to metabolism and chemical toxicity. Chem Res Toxicol. 14, 611-50; Pfeifer, G.P. et al. (2002) Tobacco smoke carcinogens, DNA damage and p 53 mutations in smoking-associated cancers. Oncogene. 21, 7435-7451]. The conversion of polycyclic aromatic hydrocarbons (PAHs), also present in cigarette smoke (CS), can lead to the production of carcinogenic intermediates that can interact with genomic DNA [Kim, J.H. et al. (1998) Metabolism of benzo[a]pyrene and benzo[a]pyrene-7,8-diol by human cytochrome P450 1B1. Carcinogenesis. 19, 1847-53]. These interactions contribute to the formation of DNA adducts and when unrepaired can lead to mutations [Piipari, R. et al. (2000) Expression of CYP1A1, CYP1B1 and CYP3A, and polycyclic aromatic hydrocarbon-DNA adduct formation in bronchoalveolar macrophages of smokers and non-smokers. Int J Cancer. 86, 610-6; Phillips, D.H. et al. (1990) Influence of cigarette smoking on the levels of DNA adducts in human bronchial epithelium and white blood cells. Int J Cancer. 46, 569-75]. Various genes encoding xenobiotic metabolizing enzymes, such as genes encoding CYP and glutathione S-transferase (GST) families, are overexpressed in response to CS exposure [Kim, J.H. et al. (1998) Metabolism of benzo[a]pyrene and benzo[a]pyrene-7,8-diol by human cytochrome P450 1B1. Carcinogenesis. 19, 1847-53; Chari, R. et al. (2007) Effect of active smoking on the human bronchial epithelium transcriptome. BMC Genomics. 8, 297; Spira, A. et al. (2004) Effects of cigarette smoke on the human airway epithelial cell transcriptome. Proc Natl Acad Sci USA. 101, 10143-8; Sutter, T.R. et al. (1994) Complete cDNA sequence of a human dioxin-inducible mRNA identifies a new gene subfamily of cytochrome P450 that maps to chromosome 2. J Biol Chem. 269, 13092-9; Shimada, T. et al. (1996) Activation of chemically diverse procarcinogens by human cytochrome P-450 1B1. Cancer Res. 56, 2979-84; Fukumoto, S. et al. (2005) Overexpression of the aldo-keto reductase family protein AKR1B10 is highly correlated with smokers' non-small cell lung carcinomas. Clin Cancer Res. 11, 1776-85; Piipari, R. et al. (2003) Glutathione S-transferases and aromatic DNA adducts in smokers' bronchoalveolar macrophages. Lung Cancer. 39, 265-72; Beane, J. et al. (2011) Characterizing the impact of smoking and lung cancer on the airway transcriptome using RNA-Seq. Cancer Prev Res (Phila). 4, 803-17], and these findings have been reproduced in smoke-exposed rodent tissues [Gebel, S. et al. (2004) Gene expression profiling in respiratory tissues from rats exposed to mainstream cigarette smoke. Carcinogenesis. 25, 169-7848; Gebel, S. et al. (2006) The kinetics of transcriptomic changes induced by cigarette smoke in rat lungs reveals a specific program of defense, inflammation, and circadian clock gene expression. Toxicol Sci. 93, 422-31].

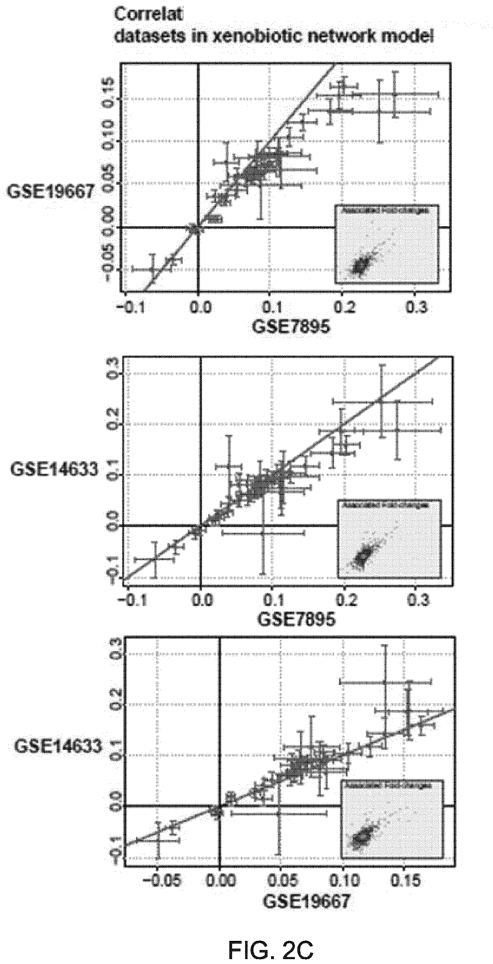

Development of xenobiotic metabolism network model The xenobiotic metabolism network model is a sub-network of the Cellular Stress Network [Schlage, W.K. et al. (2011) A computable cellular stress network model for non-diseased pulmonary and cardiovascular tissue. BMC SystBiol. 5, 168], where the transcriptional activity of aryl hydrocarbon receptor (taof(Ahr)) is central. Ahr is a transcription factor that is activated by xenobiotics and regulates the expression of target genes (e.g., exp(Cyp1A1)). The original network published by Schlage et al. [Schlage, W.K. et al. (2011) A computable cellular stress network model for non-diseased pulmonary and cardiovascular tissue. BMC SystBiol. 5, 168] may be updated to more accurately represent processes occurring in the lung and airways. The nodes and edges of the current xenobiotic metabolism network are shown below in the context of a use case (FIG. 2C).

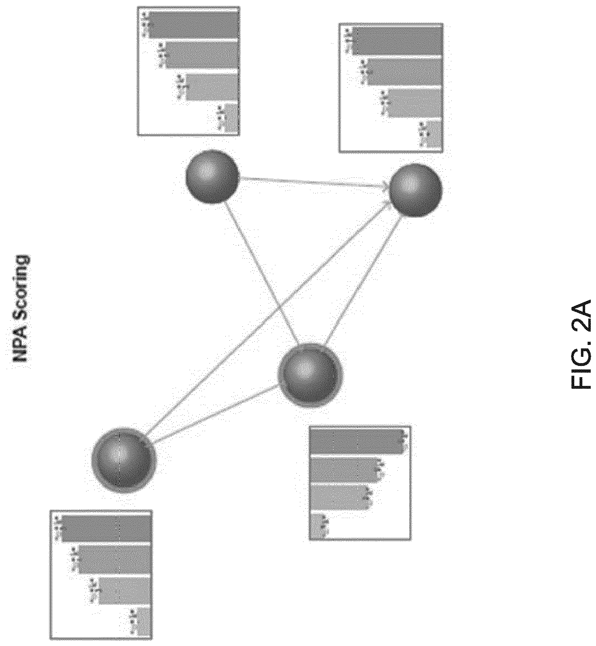

Network Perturbation Amplitude (NPA) Scoring The biological network models described herein can be used on any relevant dataset to gain a qualitative overview of the biological processes that are affected by a given stimulus. A computational approach of the disclosure enables a quantitative measure of the perturbation of biological processes [Martin, F. et al. (2012) Assessment of network perturbation amplitude by applying high-throughput data to causal biological networks. BMC Syst Biol. 6, 54]. The new network perturbation amplitude (NPA), presented here, overcomes some limitations and assumptions of previously known algorithms [Martin, F. et al. (2012) Assessment of network perturbation amplitude by applying high-throughput data to causal biological networks. BMC Syst Biol. 6, 54], in particular by integrating the network topology as well as the directionality and sign of the edges in the computation. FIG. 2A illustrates network perturbation amplitude scores assessed by mathematical transformation of differentially expressed genes using a network model. In addition to the confidence intervals of the NPA scores that account for experimental error, companion statistics derived to inform on the specificity of the NPA score with respect to the biology described in the network are shown as *O and k*, respectively, if the corresponding $p$-values are below the significance level (here 0.05). Derivation of the companion statistics (known as the O-statistic and the K-statistic) is described in more detail in relation to FIGS. 6 and 7.

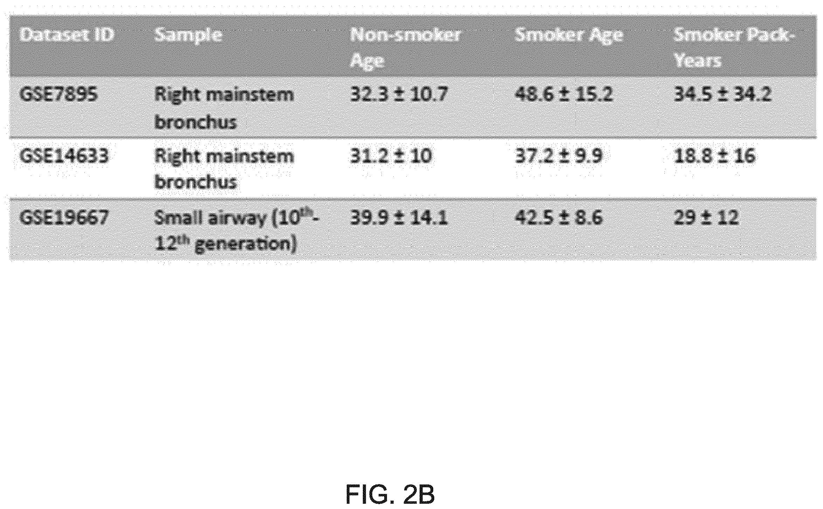

Analysis of transcriptome data using the network approach To demonstrate the validity of the NPA approach, the xenobiotic metabolism network was compared to three *in vivo* datasets from airway brushings. FIG. 2B illustrates *in vivo* bronchial brushing datasets that were scored against a xenobiotic metabolism network model. Although large individual variation is expected in human populations, the xenobiotic metabolism network can reproducibly capture perturbation in all three datasets. FIG. 2C illustrates a comparison of human bronchial brushing datasets in the context of a xenobiotic metabolism network (i-ii). Each data point represents a node in the network. The inset in each graph illustrates the comparability of the gene expressions under the nodes in the network. The differential expression levels of genes downstream of nodes in the network are somewhat comparable (insets in FIG. 2C); however, the scores on the network node are highly aligned.

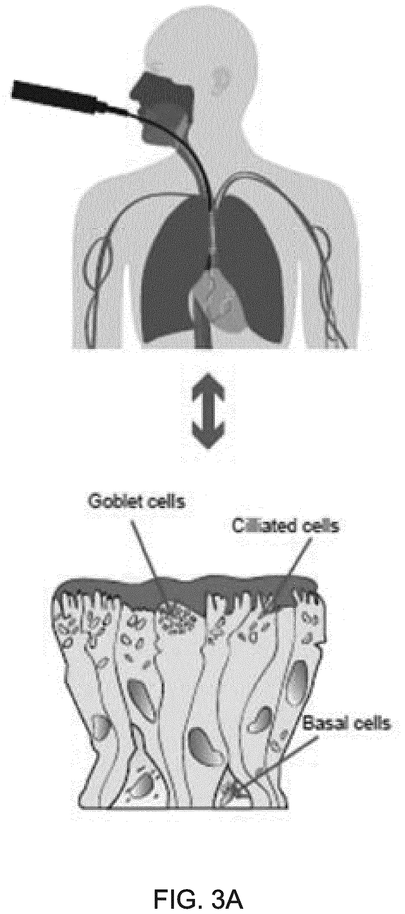

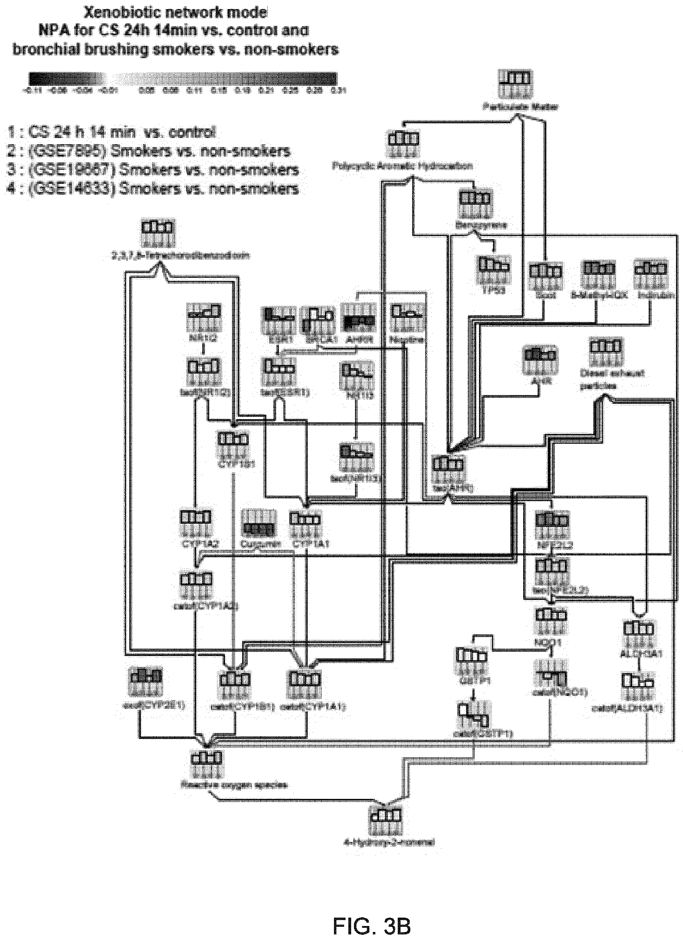

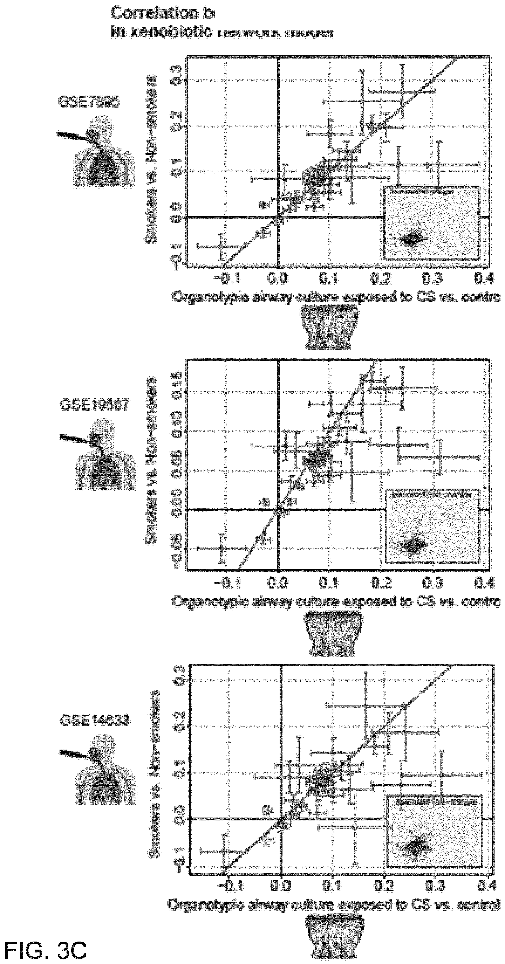

Current research focuses on relevant and robust *in vitro* culture systems to mimic smoke exposure in human subjects. An *in vitro* organotypic human-derived tracheal/bronchial epithelial pseudo-stratified culture closely resembles the epithelial tissue of the human respiratory tract both morphologically and at a molecular level [Bosse, Y. et al. (2012) Molecular Signature of Smoking in Human Lung Tissues. Cancer Res. 72, 3753-3763; Karp, P.H. et al. (2002) An in vitro model of differentiated human airway epithelia. Methods Mol. Biol. 188, 115-137; Mathis, C. et al. (2013) Human bronchial epithelial cells exposed in vitro to cigarette smoke at the air-liquid interface resemble bronchial epithelium from human smokers. American Journal of Physiology-Lung Cellular and Molecular Physiology; Maunders, H. et al. (2007) Human bronchial epithelial cell transcriptome: gene expression changes following acute exposure to whole cigarette smoke in vitro. Am J Physiol Lung Cell Mol Physiol. 292, L1248-56; Pezzulo, A. A. et al. (2011) The air-liquid interface and use of primary cell cultures are important to recapitulate the transcriptional profile of in vivo airway epithelia. Am J Physiol Lung Cell Mol Physiol. 300, L25-31]. In the previous study, a translatable mechanism was identified between bronchial brushings from smokers and organotypic *in vitro* culture of normal human bronchial epithelial cells exposed to mainstream CS for 14 min at the air-liquid interface with post-exposure of 24 hours [Mathis, C. et al. (2013) Human bronchial epithelial cells exposed in vitro to cigarette smoke at the air-liquid interface resemble bronchial epithelium from human smokers. American Journal of Physiology-Lung Cellular and Molecular Physiology] (FIG. 3A). The aim of this case was to compare the xenobiotic metabolism scores between the transcriptomic data from the *in vivo* bronchial brushing with the organotypic *in vitro* culture of normal human bronchial epithelial cells exposed to mainstream CS for 14 min at the air-liquid interface with post-exposure of 24 hours. These analyses can be extended to use the xenobiotic metabolism network model as a readout for CS exposure. FIG. 3B illustrates an assessment of three *in vivo* datasets and an *in vitro* dataset in the context of a xenobiotic metabolism network. FIG. 3C shows individual comparisons of the *in vivo* datasets to the *in vitro* dataset in the context of a xenobiotic metabolism network model. (i-iii). Each data point represents a node in the network. The inset in each graph illustrates the comparability of the gene expressions under the nodes in the network. FIGS. 3B and 3C show similar activation of the nodes in the network in smoker bronchial brushings and the *in vitro* system exposed to CS. By contrast, there is no obvious correlation in the differential expression of genes downstream of the nodes in the network (insets in FIGS. 2B and 2C). In summary, this disclosure demonstrates that the comparability *of in vivo* to *in vitro* situations at the network node level is superior to the comparability at the level of differentially expressed genes.

**Case 2: Mechanistic approach to the quantification of perturbed processes in the lungs of C57/Bl6 mice following CS exposure and cessation**

Chronic Obstructive Pulmonary Disease (COPD) is the major cause of chronic morbidity and mortality in the world [Mathers, C.D. and Loncar, D. (2006) Projections of global mortality and burden of disease from 2002 to 2030. PLoS medicine. 3, e442]. C57/Bl6 mice exposed to CS provides valuable insight into emphysema initiation and progression [Churg, A. Cosio, M. and Wright, J.L. (2008) Mechanisms of cigarette smoke-induced COPD: insights from animal models. American Journal of Physiology-Lung Cellular and Molecular Physiology. 294, L612-L631], although this mimics only some aspects of early human COPD, characterized by reduced lung function, abnormal inflammatory response in the airways, small airway remodeling, and the destruction of lung alveolar tissue [Pauwels, R.A. and Rabe, K.F. (2004) Burden and clinical features of chronic obstructive pulmonary disease (COPD). Lancet. 364, 613-20; Rabe, K.F. et al. (2007) Global strategy for the diagnosis, management, and prevention of chronic obstructive pulmonary disease: GOLD executive summary. Am J Respir Crit Care Med. 176, 532-55].

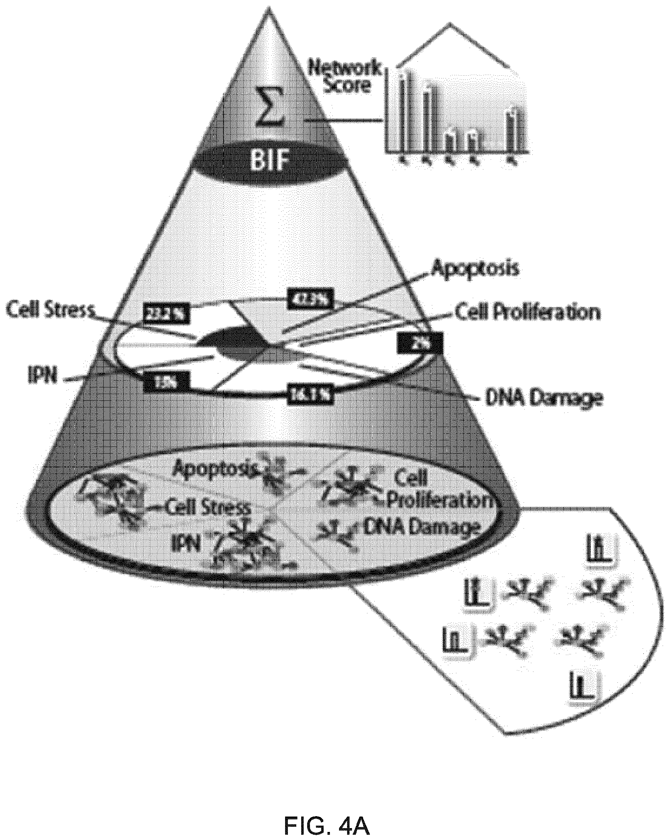

TheBiologicalImpactFactor In addition to NPA, the Biological Impact Factor (BIF) algorithm was used for this use case. BIF is an aggregated quantity for the biological impact resulting from the exposure of a biological system to one or several stimuli. In particular, BIF provides a systems-wide score for all of the processes that are captured in the network models and their associated NPA scores. FIG. 4A shows a biological impact factor cone illustrating various networks and corresponding individual perturbation amplitudes, showing the contribution of the various mechanisms to the overall biological impact factor.

Analysis of transcriptome data using the network approach The mechanism-based systems toxicology strategy [Hoeng, J. et al. (2012) A network-based approach to quantifying the impact of biologically active substances. Drug Discov Today. 17, 413-8] can be applied to gain mechanistic insights and quantify the various biological processes activated in smoke- exposed mouse lungs. In particular, transcriptomic data was obtained from mice exposed to mainstream CS for 2, 3, and 7 months as well as data after 2 and 3 months smoke exposure followed by a cessation period of 2 month and 5 months, respectively. Similar to the findings from a study by Beckett et al. [Beckett, E.L. et al. (2013) A new short-term mouse model of chronic obstructive pulmonary disease identifies a role for mast cell tryptase in pathogenesis. Journal of Allergy and Clinical Immunology], the mice developed emphysema in the lungs just after 2 months of CS exposure, characterized by lung morphometry and histopathological analysis.

The overall Biological Impact Factor (BIF) was computed using the Pulmonary Inflammatory Processes Network (IPN), Cell Proliferation Network [Westra, J.W. et al. (2011) Construction of a computable cell proliferation network focused on non-diseased lung cells. BMC Syst Biol. 5, 105], Cell Stress Network [Schlage, W.K. et al. (2011) A computable cellular stress network model for non-diseased pulmonary and cardiovascular tissue. BMC Syst Biol. 5, 168] and the networks that constitute the DNA damage, Autophagy, Cell death (apoptosis and necroptosis) and Senescence Network (DACS) [Gebel, S. et al. (2013) Construction of a Computable Network Model for DNA Damage, Autophagy, Cell Death, and Senescence Bioinformatics and Biology Insights 7, 97-117]. FIG. 4A shows the relative BIF of CS-exposed versus sham-exposed mouse lungs at each time point. The impact of CS on biological processes in the lung is at its highest after 3 month of CS exposure and is significantly decreased after cessation. Similar to endpoints related to lung function and pathology, the extent of impact depends on both the duration of smoke exposure and length of cessation. Remarkably, when mice are switched to fresh air after 2 months of CS exposure, the relative BIF drops to an almost negligible level by month 7, indicating almost complete reversal of molecular changes in the lung.

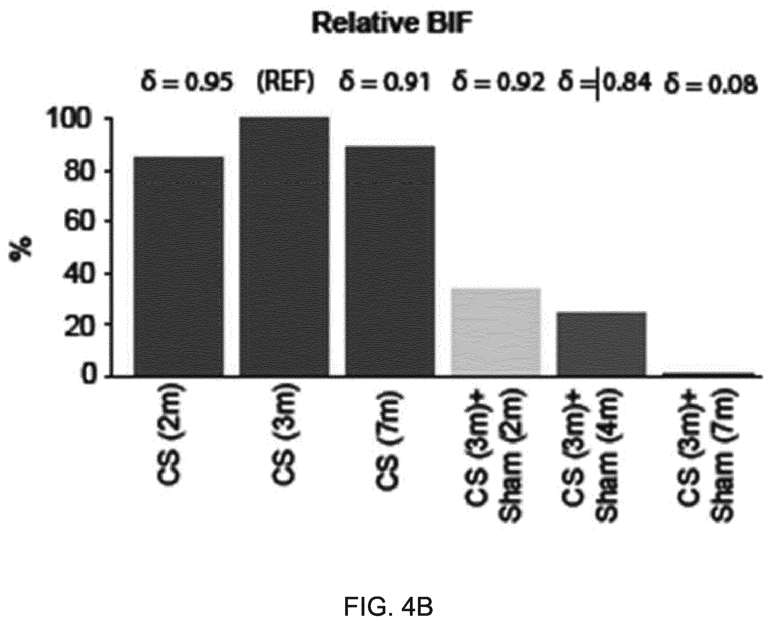

FIG. 4B shows relative biological impact factor data for smoke-exposed mouse lungs using a Pulmonary Inflammatory Processes Network (IPN), Cell Proliferation Network, Cell Stress Network and sub networks that constitute DNA damage, Autophagy, Cell death (apoptosis and necrosis) and Senescence network (DACS). The barplot in FIG. 4B shows BIF values relative to the maximum response group (REF). The $\delta$ values (-1 to 1) indicate how similar the underlying network perturbations are with respect to the maximum response group (REF). Scores were computed using transcriptomic profiling data from CS and cessation mice compared to time-matched control mice. In general, decomposing the BIF into its individual network models can identify the biological processes that most contribute to the disease phenotype at each time point. In accordance with both pathological and molecular endpoints measured in bronchoalveolar lavage fluid (BALF), lung inflammation is the predominant response to CS at all time points. Interestingly, while inflammation seems to subside after 5 month of cessation following 2 months of CS exposure, apoptosis and proliferation are still present. This is likely to reflect the ongoing repair processes in the damaged lung that seemed to have partially recovered from CS exposure as determined by histological analysis.

FIG. 4C illustrates starplots showing a decomposition of the overall relative BIF into its main mechanistic components (from cell proliferation to inflammation) for various treatment groups. The labels in a given treatment group show the contribution (in percentages) of each network for that particular treatment. They sum to 100%. The starplots have eight axes, and the legend at the right-hand side of Fig. 4C lists eight colour-coded components. These are arranged in each starplot in a counter-clockwise direction beginning with cell proliferation in the sector aligned on the vertically upwards axis (i.e. north), through cell stress on the upper left (northwest) axis, and so on, to IPN on the upper right (northeast) axis, so that in the top-left starplot (for CS(2m)) the eight components have values of 3% (cell proliferation), 3.7% (cell stress), 3.1% (apoptosis), 0.6% (autophagy), 0% (DNA damage), 0% (necroptosis), 11.4% (senescence) and 78.2% (IPN); each of the other plots has the same arrangement.

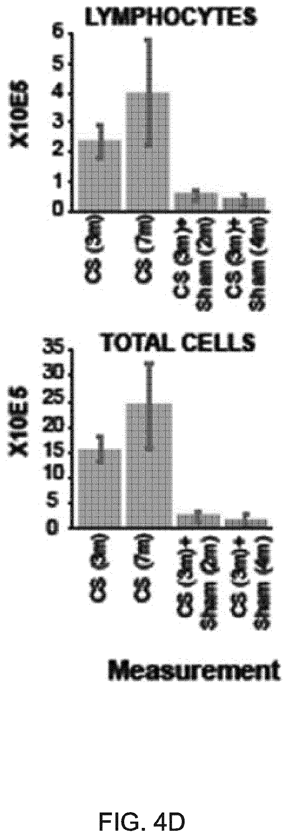

FIG. 4D is a graphical representation of Bronchoalveolar lavage fluid (BALF) cell counts from smoke-exposed mice. Cell measures are reported in number per lung. When the network BIF and lung inflammation in particular, are compared to cell counts in BALF, there is a clear concordance; CS exposure results in inflammatory cell influx into the bronchoalveolar space, and this effect is diminished upon cessation. This demonstrates that the BIF is very sensitive in capturing inflammatory changes in the lung upon smoke exposure and well reflects the recovery following the cessation period.

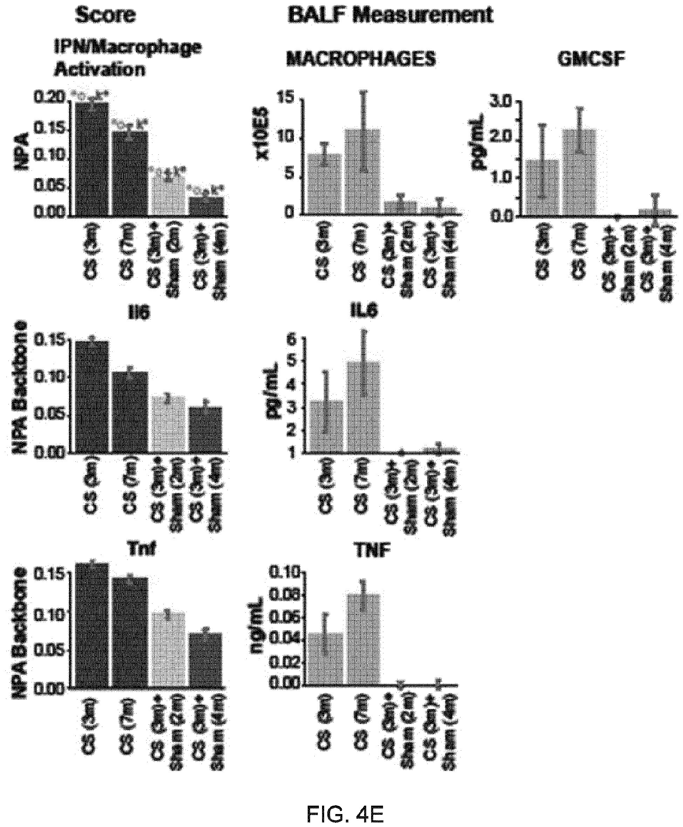

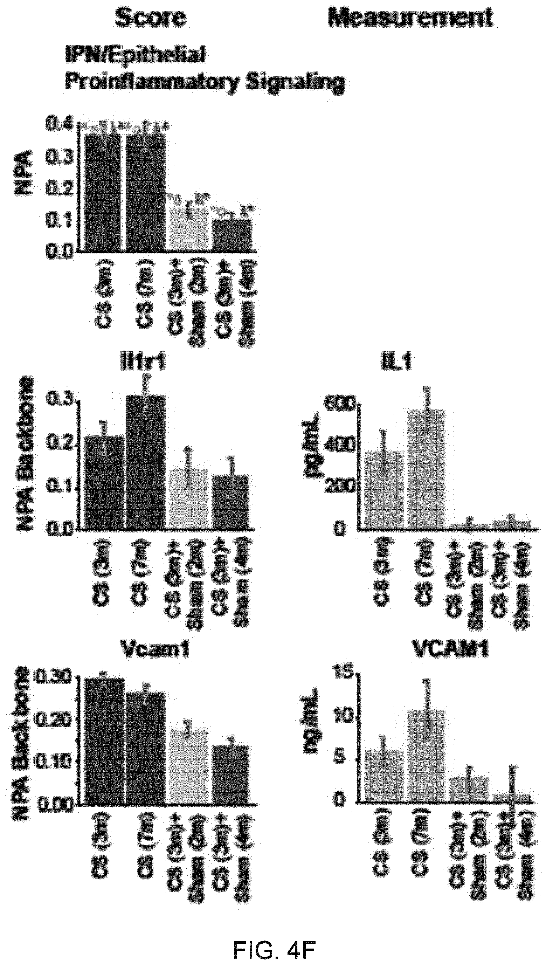

FIG. 4E is a graphical representation of NPA calculations for cell type-specific subnetworks within the Pulmonary Inflammatory Processes Network (IPN) and corresponding measurements from BALF for macrophage activation, and FIG. 4E is a graphical representation of NPA calculations for cell type-specific subnetworks within the Pulmonary Inflammatory Processes Network (IPN) and corresponding measurements from BALF for epithelial proinflammatory signaling. In both FIGS. 4E and 4F, Pigmented macrophage measures are reported in arbitrary units. Ligand levels are reported in pg/mL. Error bars represent 95% confidence intervals.

The pulmonary IPN consists of several different cell type-specific subnetworks, and how macrophage activation (FIG. 4E) and epithelial proinflammatory signaling subnetworks (FIG. 4F) are perturbed in smoke-exposed mouse lungs may be dissected. For overall macrophage activation, there is concordance between the subnetwork activation and changes in the number of BALF cells (FIG. 4E). In a mechanistic breakdown of the aggregated scores, the individual perturbation of each network node can be investigated. Examples of typical network node scores and their corresponding BALF analyte measurements can be shown for each subnetwork model to compare the behavior of primary tissue signaling to its surrogate measurement. The trends are consistent between the node scores in the network models and the BALF analytes (FIGS. 4E and 4F). Interestingly, in many cases, when the BALF measurements indicate that the inflammation has subsided upon cessation, increased NPA scores indicate that the network is still perturbed. This underscores the validity and sensitivity of the network approach, which provides mechanistic insight into disease progression and captures any residual signaling in the primary tissue that is not necessarily reflected in surrogate measurements (BALF analytes). The residual signal could be because of the presence of aggregated macrophages and other inflammatory cells (termed macrophage nests) in the alveolar space that are not necessarily reflected in the BALF compartment [W. Stinn, A. et al. (2012) Lung inflammatory effects, tumorigenesis, and emphysema development in a long-term inhalation study with cigarette mainstream smoke in mice. Toxicol. Sci. 305:49-64]. Although the correlation to a surrogate measurement serves as a proof-of-principle for our approach, the mechanistic insight can be used to develop a testable hypothesis that allows for more clever experimental design.

This disclosure describes an ability to gain quantitative systems-level mechanistic insight into the effects of exposures (e.g., biologically active substances and environmental insults) has a variety of practical applications that range from drug development to consumer safety. For example, candidate compounds can be screened for their effects on therapeutically relevant signaling pathways (e.g., inhibition of cell cycle), or molecular mechanisms modulated by chemical exposure can be quantitatively evaluated for their possible association with health risk (e.g., induction of DNA damage). Both of these examples highlight the pressing need to assess the biological impact of exposure, whether the ultimate goal is a therapeutic intervention or harm reduction.

In accordance with an implementation of the disclosure, five biological network models are constructed in the lung context [Schlage, W.K. et al. (2011) A computable cellular stress network model for non-diseased pulmonary and cardiovascular tissue. BMC SystBiol. 5, 168; Westra, J.W. et al. (2011) Construction of a computable cell proliferation network focused on non-diseased lung cells. BMC SystBiol. 5, 105; Gebel, S. et al. (2013) Construction of a Computable Network Model for DNA Damage, Autophagy, Cell Death, and Senescence Bioinformatics and Biology Insights 7, 97-117]. These network models provide an ideal substrate for creating pulmonary disease models, such as Chronic Obstructive Pulmonary Disease (COPD) or cystic fibrosis, and as described herein, reach their full power when combined with algorithms that can quantitate the perturbation of the modeled biological processes.

The systems and methods of the present disclosure evaluate the biological impact of exposures in an objective, systematic, and quantifiable manner, enabling the computation of a systems-wide and pan-mechanistic biological impact measure for a given active substance or mixture. The results described herein suggest that various fields of human disease research, from drug development to consumer product testing and environmental impact analysis, could benefit from using this methodology.

**Claims**

1. A computerized method for quantifying a perturbation of a biological system in response to an agent, comprising:

receiving, at a processing circuitry, a set of treatment data corresponding to a response of the biological system to the agent, wherein the biological system includes a plurality of biological entities, each biological entity interacting with at least one other of the biological entities;
receiving a set of control data corresponding to a response of the biological system not exposed to the agent;
wherein the set of treatment data and the set of control data both correspond to one of measurements for gene expression, protein expression, microRNA expression, post-translational modification measurements, protein modification measurements, translocation measurements, or antibody production metabolite profiles;
identifying a first computational causal network model comprising a first subset of the plurality of biological entities that represents a first biological process in the biological system based on a first biological mechanism to be characterized;
identifying a second computational causal network model comprising a second subset of the plurality of biological entities that represents a second biological process in the biological system based on a second biological mechanism to be characterized;
computing, at the processing circuitry and using the set of treatment data and the set of control data, a first network perturbation amplitude (NPA) score for the first computational causal network model that represents a first perturbation of the first subset of the plurality of biological entities in response to the agent, the first NPA score expressing a magnitude of changes within the first subset of biological entities;
computing, at the processing circuitry and using the set of treatment data and the set of control data, a second NPA score for the second computational causal network model that represents a second perturbation of the second subset of the plurality of biological entities in response to the agent, the second NPA score expressing a magnitude of changes within the second subset of biological entities; and
generating a factor based on the first NPA score, the second NPA score, and an intersection between the first computational causal network model and the second computational causal network model by aggregating the first score and the second score to obtain an aggregate score and adjusting the aggregate score to reflect the effect of the intersection on the aggregated score by computing a scalar product that comprises a non-orthogonal direct sum between the first and second computational causal network models, the non-orthogonal direct sum accounting for the intersection;
wherein the intersection includes a third subset of the plurality of biological entities, each biological entity in the third subset belonging to the first subset and the second subset, the factor representing the perturbation of the biological system in response to the agent.

2. The computerized method of claim 1, further comprising determining a statistical significance of the first NPA score to assess a specificity of the first NPA score with respect to the first computational causal network model by:

modifying the first computational causal network model N times to generate N test models;
computing a test score for each of the N test models, using the set of treatment data and the set of control data, to obtain N test scores; and
comparing the first NPA score to the N test scores, such that the first NPA score is determined to be statistically significant if the first NPA score exceeds a predetermined threshold percentage of the N test scores.

3. The computerized method of claim 2, wherein each biological entity in the first computational causal network model is a gene represented by a label on a node, and the modifying includes randomly reassigning the labels on the nodes within the first computational causal network model.

4. The computerized method of claim 2, wherein:

the first computational causal network model further includes a set of edges, each edge connecting two biological entities in the first set, and

the modifying includes randomly reassigning each edge in the set of edges to be positioned between two other biological entities in the first set.

5. The computerized method of claim 4, wherein:

n of the edges in the set of edges indicate a negative relationship between two biological entities in the first set, m of the edges in the set of edges indicate a positive relationship between two biological entities in the first set, and the randomly reassigning each respective edge is performed without regard to whether the respective edge indicates the negative relationship or the positive relationship.

6. The computerized method of claim 1, wherein each of the first computational causal network model and the second computational causal network model is representative of a biological mechanism in the biological system.

7. The computerized method of claim 1, wherein the computing the first NPA score comprises computing Sobolev-type semi-norm on a signed directed graph underlying the first computational causal network model, where the signed directed graph includes nodes for the biological entities in the first subset of the plurality of biological entities and edges connecting pairs of biological entities.

8. The computerized method of claim 7, wherein:

the computing the semi-norm comprises generating an adjacency matrix, wherein each respective element in the adjacency matrix indicates a sign of an edge between a respective pair of biological entities in the first subset, the sign being negative if the edge indicates a negative relationship between the respective pair of biological entities, and the sign being positive if the edge indicates a positive relationship between the respective pair of biological entities.

9. The computerized method of claim 7, wherein:

the first score semi-norm is computed by computing a quadratic form of a vector f and a matrix Q, wherein the vector f includes activity values representative of a difference between the set of treatment data and the set of control data, and the matrix Q is computed by assessing a first diagonal matrix representative of outgoing edges that exit the nodes in the signed directed graph and a second diagonal matrix representative of incoming edges that enter the nodes in the signed directed graph.

10. The computerized method of claim 9, wherein the matrix Q is computed by summing the first diagonal matrix and the second diagonal matrix.

11. The computerized method of claim 9, wherein:

the matrix Q is computed by subtracting a metric from a sum of the first diagonal matrix and the second diagonal matrix, the metric being based at least in part on an adjacency matrix, wherein each respective element in the adjacency matrix indicates a sign of an edge between a respective pair of biological entities in the first subset, the sign being negative if the edge indicates a negative relationship between the respective pair of biological entities, and the sign being positive if the edge indicates a positive relationship between the respective pair of biological entities.

12. The computerized method of claim 11, wherein the adjacency matrix is a weighted matrix, such that each respective element in the adjacency matrix is weighted by a value associated with a number of corresponding downstream nodes relative to the element.

13. The computerized method of claim 1, wherein obtaining the aggregated score comprises calculating a direct sum between the first and second computational causal network models and wherein adjusting the aggregate score comprises cancelling the effect of the intersection from the aggregate score, such that the adjusted aggregate score cancels a duplicative contribution of the intersection of the first computational causal network model and the second computational causal network model.

14. The computerized method of claim 13, wherein the scalar product is:

$$\sum_i w_i \|f_i\|^2_{l2(V_i)} - 1/m \sum_{i,j;i \neq j} w_i w_j < \theta_i^j(f_i) | \theta_j^i(f_j) >$$

wherein $w_i$ is a positive weight associated with a computational causal network model $i$, $f_i$ is a backbone score for the computational causal network model i, m is the number of computational causal network models in the non-orthogonal sum, and $\theta_i^j$ is a restriction operator that sets $< \theta_i^j(f_i) | \theta_j^i(f_j) >$ to zero for orthogonal components between the computational causal network model $i$ and a computational causal network model $j$, where $i$ sums over the first computational causal network model and the second computational causal network model.

15. A computerized system comprising a processing device configured with non-transitory computer-readable instructions that, when executed, cause the processing device to carry out the method of any of claims 1-14.

**Patentansprüche**

1. Computergestütztes Verfahren zur Quantifizierung einer Störung eines biologischen Systems in Reaktion auf einen Wirkstoff, umfassend:

Empfangen eines einer Reaktion des biologischen Systems auf den Wirkstoff entsprechenden Satzes von Behandlungsdaten in einer Verarbeitungsschaltung, wobei das biologische System eine Vielzahl biologischer Entitäten beinhaltet, wobei jede biologische Entität mit wenigstens einer anderen der biologischen Entitäten interagiert;
Empfangen eines Satzes von einer Reaktion des dem Wirkstoff nicht ausgesetzten biologischen Systems entsprechenden Kontrolldaten;
wobei der Satz von Behandlungsdaten und der Satz von Kontrolldaten beide jeweils einer der Messungen für Genexpression, Proteinexpression, mikroRNA-Expression, posttranslationale Modifikationsmessungen, Proteinmodifikationsmessungen, Translokationsmessungen oder Antikörperproduktionsmetabolitprofilen entsprechen;
Identifizieren eines ersten rechnerischen kausalen Netzmodells, das eine erste Teilmenge der Vielzahl biologischer Entitäten umfasst, die einen ersten biologischen Prozess in dem biologischen System basierend auf einem ersten zu charakterisierenden biologischen Mechanismus darstellt;
Identifizieren eines zweiten rechnerischen kausalen Netzmodells, das eine zweite Teilmenge der Vielzahl biologischer Entitäten umfasst, die einen zweiten biologischen Prozess in dem biologischen System basierend auf einem zweiten zu charakterisierenden biologischen Mechanismus darstellt;
Berechnen, in der Verarbeitungsschaltung und unter Verwendung des Satzes von Behandlungsdaten und des Satzes von Kontrolldaten, eines ersten Wertes der Netzwerkstörungsamplitude (NPA) für das erste rechnerische kausale Netzmodell, das eine erste Störung der ersten Teilmenge der Vielzahl biologische Entitäten in Reaktion auf den Wirkstoff darstellt, wobei der erste NPA-Wert eine Größe von Änderungen innerhalb der ersten Teilmenge biologischer Entitäten ausdrückt;
Berechnen, in der Verarbeitungsschaltung und unter Verwendung des Satzes von Behandlungsdaten und des Satzes von Kontrolldaten, eines zweiten NPA-Wertes für das zweite rechnerische kausale Netzmodell, das eine zweite Störung der zweiten Teilmenge der Vielzahl biologischer Entitäten in Reaktion auf den Wirkstoff darstellt, wobei der zweite NPA-Wert eine Größe von Änderungen innerhalb der zweiten Teilmenge biologischer Entitäten ausdrückt; und
Erzeugen eines Faktors basierend auf dem ersten NPA-Wert, dem zweiten NPA-Wert und einer Überschneidung zwischen dem ersten rechnerischen kausalen Netzmodell und dem zweiten rechnerischen kausalen Netzmodell durch Aggregieren des ersten Wertes und des zweiten Wertes zum Erhalten eines aggregierten Wertes und Anpassen des aggregierten Wertes, um die Auswirkung der Überschneidung auf den aggregierten Wert durch Berechnen eines Skalarprodukts widerzuspiegeln, das eine nicht-orthogonale direkte Summe zwischen dem ersten und dem zweiten rechnerischen kausalen Netzmodell aufweist, wobei die nicht-orthogonale direkte Summe die Überschneidung berücksichtigt;

wobei die Überschneidung eine dritte Teilmenge der Vielzahl biologischer Entitäten beinhaltet, jede biologische Entität in der dritten Teilmenge zu der ersten Teilmenge und der zweiten Teilmenge gehört und der Faktor die Störung des biologischen Systems in Reaktion auf den Wirkstoff darstellt.

2. Computergestütztes Verfahren nach Anspruch 1, ferner umfassend das Ermitteln einer statistischen Signifikanz des ersten NPA-Wertes zur Bewertung einer Spezifität des ersten NPA-Wertes in Bezug auf das erste rechnerische kausale Netzmodell durch:

N-faches Modifizieren des ersten rechnerischen kausalen Netzmodells zur Erzeugung von N Testmodellen; Berechnen eines Testwertes für jedes der N Testmodelle unter Verwendung des Satzes von Behandlungsdaten und des Satzes von Kontrolldaten zum Erhalt von N Testwerten; und Vergleich des ersten NPA-Wertes mit den N Testwerten, sodass der erste NPA-Wert als statistisch signifikant ermittelt wird, wenn der erste NPA-Wert einen vorbestimmten Schwellenprozentsatz der N Testwerte überschreitet.

3. Computergestütztes Verfahren nach Anspruch 2, wobei jede biologische Entität in dem ersten rechnerischen kausalen Netzmodell ein Gen ist, das durch eine Markierung an einem Knoten dargestellt wird, und das Modifizieren eine zufällige Neuzuweisung der Markierungen an den Knoten innerhalb des ersten rechnerischen kausalen Netzmodells beinhaltet.

4. Computergestütztes Verfahren nach Anspruch 2, wobei:

das erste rechnerische kausale Netzmodell ferner einen Satz von Kanten beinhaltet, wobei jede Kante zwei biologische Entitäten in dem ersten Satz verbindet, und das Modifizieren eine zufällige Neuzuweisung jeder Kante in dem Satz von Kanten zur Positionierung zwischen zwei anderen biologischen Entitäten in dem ersten Satz beinhaltet.

5. Computergestütztes Verfahren nach Anspruch 4, wobei:

n der Kanten in dem Satz von Kanten eine negative Beziehung zwischen zwei biologischen Entitäten in dem ersten Satz anzeigen, m der Kanten in dem Satz von Kanten eine positive Beziehung zwischen zwei biologischen Entitäten in dem ersten Satz anzeigen, und die zufällige Neuzuweisung jeder einzelnen Kante ohne Berücksichtigung dessen durchgeführt wird, ob die jeweilige Kante die negative Beziehung oder die positive Beziehung anzeigt.

6. Computergestütztes Verfahren nach Anspruch 1, wobei das erste rechnerische kausale Netzmodell und das zweite rechnerische kausale Netzmodell jeweils einen biologischen Mechanismus in dem biologischen System darstellen.

7. Computergestütztes Verfahren nach Anspruch 1, wobei die Berechnung des ersten NPA-Wertes die Berechnung der Halbnorm vom Sobolev-Typ auf einem signierten gerichteten Graphen aufweist, der dem ersten rechnerischen kausalen Netzmodell zugrunde liegt, wobei der signierte gerichtete Graph Knoten für die biologischen Entitäten in der ersten Teilmenge der Vielzahl biologischer Entitäten und Kanten beinhaltet, die Paare von biologischen Entitäten verbinden.

8. Computergestütztes Verfahren nach Anspruch 7, wobei:

die Berechnung der Halbnorm die Erzeugung einer Adjazenzmatrix umfasst, wobei jedes jeweilige Element in der Adjazenzmatrix ein Vorzeichen einer Kante zwischen einem jeweiligen Paar biologischer Entitäten in der ersten Teilmenge angibt, das Vorzeichen negativ ist, wenn die Kante eine negative Beziehung zwischen dem jeweiligen Paar biologischer Entitäten anzeigt, und das Vorzeichen positiv ist, wenn die Kante eine positive Beziehung zwischen dem jeweiligen Paar biologischer Entitäten anzeigt.

9. Computergestütztes Verfahren nach Anspruch 7, wobei:

die erste Bewertungshalbnorm durch Berechnung einer quadratischen Form eines Vektors f und einer Matrix Q berechnet wird,
wobei der Vektor f Aktivitätswerte beinhaltet, die eine Differenz zwischen dem Satz von Behandlungsdaten und dem Satz von Kontrolldaten darstellen, und
die Matrix Q durch Bewertung einer ersten diagonalen Matrix, die ausgehende Kanten darstellt, die die Knoten in dem signierten gerichteten Graphen verlassen, und einer zweiten diagonalen Matrix, die eingehende Kanten darstellt, die die Knoten in dem signierten gerichteten Graphen betreten, berechnet wird.

10. Computergestütztes Verfahren nach Anspruch 9, wobei die Matrix Q durch Summierung der ersten Diagonalmatrix und der zweiten Diagonalmatrix berechnet wird.

11. Computergestütztes Verfahren nach Anspruch 9, wobei:

die Matrix Q durch Subtraktion einer Metrik von einer Summe der ersten Diagonalmatrix und der zweiten Diagonalmatrix berechnet wird, wobei die Metrik wenigstens zum Teil auf einer Adjazenzmatrix basiert,
wobei jedes jeweilige Element in der Adjazenzmatrix ein Vorzeichen einer Kante zwischen einem jeweiligen Paar biologischer Entitäten in der ersten Teilmenge angibt,
das Vorzeichen negativ ist, wenn die Kante eine negative Beziehung zwischen dem jeweiligen Paar biologischer Entitäten anzeigt, und
das Vorzeichen positiv ist, wenn die Kante eine positive Beziehung zwischen dem jeweiligen Paar biologischer Entitäten anzeigt.

12. Computergestütztes Verfahren nach Anspruch 11, wobei die Adjazenzmatrix eine gewichtete Matrix ist, sodass jedes jeweilige Element in der Adjazenzmatrix durch einen einer Anzahl entsprechender nachgelagerter Knoten relativ zu dem Element zugewiesenen Wert gewichtet wird.

13. Computergestütztes Verfahren nach Anspruch 1, wobei das Erhalten des aggregierten Wertes das Berechnen einer direkten Summe zwischen dem ersten und dem zweiten rechnerischen kausalen Netzmodell umfasst und wobei das Anpassen des aggregierten Wertes das Aufheben des Effekts der Überschneidung aus dem aggregierten Wert umfasst, so dass der angepasste aggregierte Wert einen duplizierenden Beitrag der Überschneidung des ersten rechnerischen kausalen Netzmodells und des zweiten rechnerischen kausalen Netzmodells aufhebt.

14. Computergestütztes Verfahren nach Anspruch 13, wobei das Skalarprodukt ist:

$$\sum_i w_i ||f_i||^2_{l2(Vi)} - 1/m \sum_{i,j;i \neq j} w_i w_j < \theta_i^j (f_i) | \theta_j^i (f_j) >$$

wobei $w_i$ eine einem rechnerischen kausalen Netzmodell $i$ zugewiesene positive Gewichtung ist, $f_i$ ein Backbone-Wert für das rechnerische kausale Netzmodell $i$ ist, m die Anzahl der rechnerischen kausalen Netzmodelle in der nicht-orthogonalen Summe ist und $\theta_i^j$ ein Restriktionsoperator ist, der $< \theta_i^j (f_i) | \theta_j^i (f_j) >$ für orthogonale Komponenten zwischen dem rechnerischen kausalen Netzmodell $i$ und einem rechnerischen kausalen Netzmodell $j$ auf Null setzt, wobei $i$ über das erste rechnerische kausale Netzmodell und das zweite rechnerische kausale Netzmodell summiert.

15. Computergestütztes System, umfassend eine mit nichtflüchtigen, computerlesbaren Anweisungen ausgelegte Verarbeitungsvorrichtung, die bei Ausführung die Verarbeitungsvorrichtung veranlassen, das Verfahren nach einem der Ansprüche 1-14 auszuführen.


**Revendications**

1. Procédé informatisé destiné à quantifier une perturbation d'un système biologique en réponse à un agent, comprenant :

la réception, au niveau de circuits de traitement, d'un ensemble de données thérapeutiques correspondant à une réponse du système biologique à l'agent, dans lequel le système biologique comporte une pluralité d'entités biologiques, chaque entité biologique interagissant avec au moins une autre des entités biologiques ;

la réception d'un ensemble de données de contrôle correspondant à une réponse du système biologique non exposé à l'agent ;

dans lequel l'ensemble de données thérapeutiques et l'ensemble de données de contrôle correspondent tous deux à l'une des mesures d'expression génétique, d'expression protéique, d'expression de microARN, de mesures de modification post-traductionnelle, de mesures de modification protéique, de mesures de translocation ou de profils de métabolites de production d'anticorps ;

l'identification d'un premier modèle de réseau de causalité computationnel comprenant un premier sous-ensemble de la pluralité d'entités biologiques qui représente un premier processus biologique dans le système biologique sur la base d'un premier mécanisme biologique à caractériser ;

l'identification d'un deuxième modèle de réseau de causalité computationnel comprenant un deuxième sous-ensemble de la pluralité d'entités biologiques qui représente un deuxième processus biologique dans le système biologique sur la base d'un deuxième mécanisme biologique à caractériser ;

le calcul, au niveau des circuits de traitement et à l'aide de l'ensemble de données thérapeutiques et de l'ensemble de données de contrôle, d'un premier score d'amplitude de perturbation de réseau (NPA) pour le premier modèle de réseau de causalité computationnel qui représente une première perturbation du premier sous-ensemble de la pluralité d'entités biologiques en réponse à l'agent, le premier score NPA exprimant une ampleur de changements dans le premier sous-ensemble d'entités biologiques ;

le calcul, au niveau des circuits de traitement et à l'aide de l'ensemble de données thérapeutiques et de l'ensemble de données de contrôle, d'un deuxième NPA pour le deuxième modèle de réseau de causalité computationnel qui représente une deuxième perturbation du deuxième sous-ensemble de la pluralité d'entités biologiques en réponse à l'agent, le deuxième score NPA exprimant une ampleur de changements dans le deuxième sous-ensemble d'entités biologiques ; et

la génération d'un facteur sur la base du premier score NPA, du deuxième score NPA, et d'une intersection entre le premier modèle de réseau de causalité computationnel et le deuxième modèle de réseau de causalité computationnel en agrégeant le premier score et le deuxième score pour obtenir un score agrégé et en ajustant le score agrégé pour refléter l'effet de l'intersection sur le score agrégé par calcul d'un produit scalaire qui comprend une somme directe non orthogonale entre les premier et deuxième modèles de réseau de causalité computationnel, la somme directe non orthogonale rendant compte de l'intersection ;

dans lequel l'intersection comporte un troisième sous-ensemble de la pluralité d'entités biologiques, chaque entité biologique dans le troisième sous-ensemble appartenant au premier sous-ensemble et au deuxième sous-ensemble, le facteur représentant la perturbation du système biologique en réponse à l'agent.

2. Procédé informatisé selon la revendication 1, comprenant en outre la détermination d'une signification statistique du premier score NPA pour évaluer une spécificité du premier score NPA par rapport au premier modèle de réseau de causalité computationnel par :

la modification du premier modèle de réseau de causalité computationnel N fois pour générer N modèles d'essai ;

le calcul d'un score d'essai pour chacun des N modèles d'essai, à l'aide de l'ensemble de données thérapeutiques et de l'ensemble de données de contrôle, pour obtenir N scores d'essai ; et

la comparaison du premier score NPA aux N scores d'essai, de sorte que le premier score NPA est déterminé comme étant statistiquement significatif si le premier score NPA dépasse un pourcentage seuil prédéterminé des N scores d'essai.

3. Procédé informatisé selon la revendication 2, dans lequel chaque entité biologique dans le premier modèle de réseau de causalité computationnel est un gène représenté par une étiquette sur un noeud, et la modification comporte la réattribution aléatoire des étiquettes sur les noeuds dans le premier modèle de réseau de causalité computationnel.

4. Procédé informatisé selon la revendication 2, dans lequel :

le premier modèle de réseau de causalité computationnel comporte en outre un ensemble de bords, chaque bord reliant deux entités biologiques dans le premier ensemble, et

la modification comporte la réattribution aléatoire de chaque bord dans l'ensemble de bords à positionner entre deux autres entités biologiques dans le premier ensemble.

**5.** Procédé informatisé selon la revendication 4, dans lequel :

n des bords dans l'ensemble de bords indiquent une relation négative entre deux entités biologiques dans le premier ensemble,
m des bords dans l'ensemble de bords indiquent une relation positive entre deux entités biologiques dans le premier ensemble, et
la réattribution aléatoire de chaque bord respectif est réalisée indépendamment du fait que le bord respectif indique la relation négative ou la relation positive.

**6.** Procédé informatisé selon la revendication 1, dans lequel chacun du premier modèle de réseau de causalité computationnel et du deuxième modèle de réseau de causalité computationnel est représentatif d'un mécanisme biologique dans le système biologique.

**7.** Procédé informatisé selon la revendication 1, dans lequel le calcul du premier score NPA comporte le calcul d'une semie-norme de type Sobolev sur un graphe orienté signé sous-jacent au premier modèle de réseau de causalité computationnel, où le graphe orienté signé comporte des noeuds pour les entités biologiques dans le premier sous-ensemble de la pluralité d'entités biologiques et des bords reliant des paires d'entités biologiques.

**8.** Procédé informatisé selon la revendication 7, dans lequel :

le calcul de la semi-norme comprend la génération d'une matrice d'adjacence,
dans lequel chaque élément respectif dans la matrice d'adjacence indique un signe d'un bord entre une paire respective d'entités biologiques dans le premier sous-ensemble,
le signe étant négatif si le bord indique une relation négative entre la paire respective d'entités biologiques, et
le signe étant positif si le bord indique une relation positive entre la paire respective d'entités biologiques.

**9.** Procédé informatisé selon la revendication 7, dans lequel :

la première semie-norme de score est calculée par calcul d'une forme quadratique d'un vecteur f et d'une matrice Q,
dans lequel le vecteur f comporte des valeurs d'activité représentatives d'une différence entre l'ensemble de données thérapeutiques et l'ensemble de données de contrôle, et
la matrice Q est calculée en évaluant une première matrice diagonale représentative des bords sortants qui quittent les noeuds dans le graphe orienté signé et une deuxième matrice diagonale représentative des bords entrants qui pénètrent les noeuds dans le graphe orienté signé.

**10.** Procédé informatisé selon la revendication 9, dans lequel la matrice Q est calculée par la somme de la première matrice diagonale et de la deuxième matrice diagonale.

**11.** Procédé informatisé selon la revendication 9, dans lequel :

la matrice Q est calculée par soustraction d'une métrique d'une somme de la première matrice diagonale et de la deuxième matrice diagonale, la métrique étant basée au moins en partie sur une matrice d'adjacence,
dans lequel chaque élément respectif dans la matrice d'adjacence indique un signe d'un bord entre une paire respective d'entités biologiques dans le premier sous-ensemble,
le signe étant négatif si le bord indique une relation négative entre la paire respective d'entités biologiques, et
le signe étant positif si le bord indique une relation positive entre la paire respective d'entités biologiques.

**12.** Procédé informatisé selon la revendication 11, dans lequel la matrice d'adjacence est une matrice pondérée, de sorte que chaque élément respectif dans la matrice d'adjacence est pondéré par une valeur associée à un nombre de noeuds aval correspondants par rapport à l'élément.

**13.** Procédé informatisé selon la revendication 1, dans lequel l'obtention du score agrégé comprend le calcul d'une somme directe entre les premier et deuxième modèles de réseau de causalité computationnel et dans lequel l'ajustement du score agrégé comprend l'annulation de l'effet de l'intersection du score agrégé, de telle sorte que le score agrégé ajusté annule une contribution duplicative de l'intersection du premier modèle de réseau de causalité computationnel et du deuxième modèle de réseau de causalité computationnel.

**14.** Procédé informatisé selon la revendication 13, dans lequel le produit scalaire est :

$$\sum_i w_i ||f_i||^2_{l2(V_i)} - 1/m \sum_{i,j;i \neq j} w_i w_j \ < \ \theta_i^j(f_i) \ | \ \theta_j^i(f_j) >$$

dans lequel wi est une pondération positive associée à un modèle de réseau de causalité computationnel *i*, $f_i$ est un score de base pour le modèle de réseau de causalité computationnel *i,* m est le nombre de modèles de réseau causalité computationnel dans la somme non orthogonale, et $\theta_i^j$ est un opérateur de restriction qui établit $<\theta_i^j(f_i) | \theta_j^i(f_j)>$ à zéro pour des composantes orthogonales entre le modèle de réseau de causalité computationnel *i* et un modèle de réseau de causalité computationnel *j,* où i fait la somme du premier modèle de réseau de causalité computationnel et du deuxième modèle de réseau de causalité computationnel.

**15.** Système informatisé comprenant un dispositif de traitement configuré avec des instructions non transitoires lisibles par ordinateur qui, lorsqu'elles sont exécutées, amènent le dispositif de traitement à effectuer le procédé selon l'une quelconque des revendications 1 à 14.

FIG. 1A

FIG. 1B

FIG. 1C

FIG. 1D

NPA Scoring

FIG. 2A

| Dataset ID | Sample | Non-smoker Age | Smoker Age | Smoker Pack-Years |
|---|---|---|---|---|
| GSE7895 | Right mainstem bronchus | 32.3 ± 10.7 | 48.6 ± 15.2 | 34.5 ± 34.2 |
| GSE14633 | Right mainstem bronchus | 31.2 ± 10 | 37.2 ± 9.9 | 18.8 ± 16 |
| GSE19667 | Small airway (10th-12th generation) | 39.9 ± 14.1 | 42.5 ± 8.6 | 29 ± 12 |

FIG. 2B

Correlat
datasets in xenobiotic network model

FIG. 2C

Goblet cells

Ciliated cells

Basal cells

FIG. 3A

Xenobiotic network model
NPA for CS 24h 14min vs. control and
bronchial brushing smokers vs. non-smokers

FIG. 3B

FIG. 3C

FIG. 4A

**Relative BIF**

$\delta = 0.95$    (REF)    $\delta = 0.91$    $\delta = 0.92$   $\delta = 0.84$   $\delta = 0.08$

FIG. 4B

FIG. 4C

FIG. 4D

FIG. 4E

FIG. 4F

500

```
┌─────────────────────────────────────────────────────────────────────────────┐
│                                                                               │
│   ┌──────────────┐   ┌──────────────────────┐   ┌──────────────────────┐      │
│   │  CPU 506     │   │  COMMUNICATIONS      │   │  INPUT/OUTPUT        │      │
│   │              │   │  INTERFACE UNIT 508  │   │  CONTROLLER 510      │      │
│   └──────────────┘   └──────────────────────┘   └──────────────────────┘      │
│                                                                               │
├───────────────────────────────────────────────────────────────────────────── │
│                                                                               │
│   ┌──────────────────────┐        ┌──────────────────────────────────────┐    │
│   │  SYSTEM MEMORY       │        │              STORAGE DEVICES         │    │
│   │                      │        │                                      │    │
│   │  ┌────────────────┐  │        │   ┌──────────────────┐               │    │
│   │  │  RAM 502       │  │        │   │  OPERATING       │               │    │
│   │  └────────────────┘  │        │   │  SYSTEM 512      │               │    │
│   │                      │        │   └──────────────────┘               │    │
│   │  ┌────────────────┐  │        │                                      │    │
│   │  │  ROM 504       │  │        │   ┌──────────────┐   ┌────────────┐   │    │
│   │  └────────────────┘  │        │   │ APPLICATION(S)│  │ DATABASE(S)│   │    │
│   │                      │        │   │    514        │  │    516     │   │    │
│   └──────────────────────┘        │   └──────────────┘   └────────────┘   │    │
│                                   └──────────────────────────────────────┘    │
│                                                                               │
└───────────────────────────────────────────────────────────────────────────── ┘
```

FIG. 5

COMPUTE FIRST NPA SCORE
BASED ON UNMODIFIED
NETWORK
602

GENERATE C RANDOM
REASSIGNMENTS FOR GENE
LABELS OF SUPPORTING
NODES
606

COMPUTE C NPA SCORES
BASED ON RANDOM
REASSIGNMENTS
610

GENERATE DISTRIBUTION OF C
NPA SCORES
612

COMPARE FIRST NPA SCORE TO
THE GENERATED DISTRIBUTION
614

FIG. 6

EP 2 989 578 B1

700

COMPUTE FIRST NPA SCORE
BASED ON UNMODIFIED
NETWORK
702

DETERMINE NUMBER OF
NEGATIVE EDGES AND
NUMBER OF POSITIVE EDGES
BETWEEN BACKBONE NODES
704

RANDOMLY CONNECT
BACKBONE NODES WITH M
POSITIVE EDGES AND N
NEGATIVE EDGES C TIMES
706

COMPUTE C NPA
SCORES BASED ON
RANDOM
REASSIGNMENTS
710

GENERATE
DISTRIBUTION OF C
NPA SCORES
712

COMPARE FIRST NPA
SCORE TO THE
GENERATED
DISTRIBUTION
614

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013034300 A
- US 61525700
- US 61527946
- US 61532972
- EP 2012061035 W
- EP 2012066557 W
- EP 2012003760 W
- US 61495824
- EP 2012061033 W

### Non-patent literature cited in the description

- **KREWSKI, D. et al.** New directions in toxicity testing. *Annu Rev Public Health.,* 2011, vol. 32, 161-78
- **BHATTACHARYA, S. et al.** Toxicity testing in the 21 century: defining new risk assessment approaches based on perturbation of intracellular toxicity pathways. *PLoS One,* 2011, vol. 6, e20887
- **KELLER, D.A. et al.** Identification and characterization of adverse effects in 21st century toxicology. *Toxicological Sciences,* 2012, vol. 126, 291-297
- Committee on Toxicity Testing Assessment of Environmental Agents. Toxicity testing in the 21st century: A vision and a strategy. N.A. Press, 2007
- **ATTERWILL, C.K. ; WING, M.G.** In vitro preclinical lead optimisation technologies (PLOTs) in pharmaceutical development. *Toxicology letters,* 2002, vol. 127, 143-151
- **LEE, E. et al.** Inferring pathway activity toward precise disease classification. *PLoS computational biology,* 2008, vol. 4, e1000217
- **HOENG, J. et al.** A network-based approach to quantifying the impact of biologically active substances. *Drug Discov Today,* 2012, vol. 17, 413-8
- **LAMB, J. et al.** The Connectivity Map: using gene-expression signatures to connect small molecules, genes, and disease. *Science Signalling,* 2006, vol. 313, 1929
- **XIANG, Y. et al.** Divergence Weighted Independence Graphs for the Exploratory Analysis of Biological Expression Data. *Journal of Health & Medical Informatics,* 2011
- **IORIO, F. et al.** Discovery of drug mode of action and drug repositioning from transcriptional responses. *Proceedings of the National Academy of Sciences,* 2010, vol. 107, 14621-14626
- **LECCA, P. ; PRIAMI, C.** Biological network inference for drug discovery. *Drug discovery today,* 2012
- **MARBACH, D. et al.** Revealing strengths and weaknesses of methods for gene network inference. *Proceedings of the National Academy of Sciences,* 2010, vol. 107, 6286-6291
- **JULIA HOENG.** A network-based approach to quantifying the impact of biologically active substances. *Drug Discovery Today,* 01 May 2012, vol. 17 (9-10), 413-418
- **BADER, G.D. ; CARY, M.P. ; SANDER, C.** Pathguide: a pathway resource list. *Nucleic Acids Research,* 2006, vol. 34, D504-D506
- **HASAN, S. et al.** Network analysis has diverse roles in drug discovery. *Drug discovery today,* 2012
- **YıLDıRıM, M.A. et al.** Drug-target network. *Nature Biotechnology,* 2007, vol. 25, 1119
- **HOPKINS, A.L.** Network pharmacology: the next paradigm in drug discovery. *Nature chemical biology,* 2008, vol. 4, 682-690
- **MARTIN, F. et al.** Assessment of network perturbation amplitude by applying high-throughput data to causal biological networks. *BMC Syst Biol,* 2012, vol. 6, 54
- **DEMIR, E. et al.** The BioPAX community standard for pathway data sharing. *Nature Biotechnology,* 2010, vol. 28, 935-942
- **KYOTO.** Encyclopedia of Genes and Genomes
- **KANEHISA, M. et al.** KEGG for integration and interpretation of large-scale molecular data sets. *Nucleic Acids Res.,* 2012, vol. 40, D109-14
- **SMOOT, M.E. et al.** Cytoscape 2.8: new features for data integration and network visualization. *Bioinformatics,* 2011, vol. 27, 431-432
- *A Language and Environment for Statistical Computing,* 2009
- **GENTLEMAN, R.** Bioinformatics and computational biology solutions using Rand Bioconductor. Springer Science+Business Media, 2005, 473

- **GENTLEMAN, R.C. et al.** Bioconductor: open software development for computational biology and bioinformatics. *Genome Biol,* 2004, vol. 5, R80
- **IRIZARRY, R.A. et al.** Exploration, normalization, and summaries of high density oligonucleotide array probe level data. *Biostatistics,* 2003, vol. 4, 249-64
- **BURCHELL, B. et al.** Substrate Specificity of Human Hepatic Udp-Glucuronosyltransferases. *Methods inenzymology,* 2005, vol. 400, 46-57
- **GUENGERICH, F.P.** Common and uncommon cytochrome P450reactions related to metabolism and chemical toxicity. *Chem Res Toxicol.,* 2001, vol. 14, 611-50
- **PFEIFER, G.P. et al.** Tobacco smoke carcinogens, DNA damage and p 53 mutations in smoking-associated cancers. *Oncogene,* 2002, vol. 21, 7435-7451
- **KIM, J.H. et al.** Metabolism of benzo[a]pyrene and benzo[a]pyrene-7,8-diol by human cytochrome P450 1B1. *Carcinogenesis,* 1998, vol. 19, 1847-53
- **PIIPARI, R. et al.** Expression of CYP1A1, CYP1B1 and CYP3A, and polycyclic aromatic hydrocarbon-DNA adduct formation in bronchoalveolar macrophages of smokers and non-smokers. *Int J Cancer.,* 2000, vol. 86, 610-6
- **PHILLIPS, D.H. et al.** Influence of cigarette smoking on the levels of DNA adducts in human bronchial epithelium and white blood cells. *Int J Cancer.,* 1990, vol. 46, 569-75
- **CHARI, R. et al.** Effect of active smoking on the human bronchial epithelium transcriptome. *BMC Genomics,* 2007, vol. 8, 297
- **SPIRA, A. et al.** Effects of cigarette smoke on the human airway epithelial cell transcriptome. *Proc Natl Acad Sci USA.,* 2004, vol. 101, 10143-8
- **SUTTER, T.R. et al.** Complete cDNA sequence of a human dioxin-inducible mRNA identifies a new gene subfamily of cytochrome P450 that maps to chromosome 2. *J Biol Chem.,* 1994, vol. 269, 13092-9
- **SHIMADA, T. et al.** Activation of chemically diverse procarcinogens by human cytochrome P-450 1B1. *Cancer Res.,* 1996, vol. 56, 2979-84
- **FUKUMOTO, S. et al.** Overexpression of the aldo-keto reductase family protein AKR1B10 is highly correlated with smokers' non-small cell lung carcinomas. *Clin Cancer Res.,* 2005, vol. 11, 1776-85
- **PIIPARI, R. et al.** Glutathione S-transferases and aromatic DNA adducts in smokers' bronchoalveolar macrophages. *Lung Cancer,* 2003, vol. 39, 265-72
- **BEANE, J. et al.** Characterizing the impact of smoking and lung cancer on the airway transcriptome using RNA-Seq. *Cancer Prev Res (Phila).,* 2011, vol. 4, 803-17
- **GEBEL, S. et al.** Gene expression profiling in respiratory tissues from rats exposed to mainstream cigarette smoke. *Carcinogenesis,* 2004, vol. 25, 169-7848
- **GEBEL, S. et al.** The kinetics of transcriptomic changes induced by cigarette smoke in rat lungs reveals a specific program of defense, inflammation, and circadian clock gene expression. *Toxicol Sci.,* 2006, vol. 93, 422-31
- **SCHLAGE, W.K. et al.** A computable cellular stress network model for non-diseased pulmonary and cardiovascular tissue. *BMC SystBiol.,* 2011, vol. 5, 168
- **SCHLAGE, W.K. et al.** A computable cellular stress network model for non-diseased pulmonary and cardiovascular tissue. *BMC SystBiol,* 2011, vol. 5, 168
- **BOSSE, Y. et al.** Molecular Signature of Smoking in Human Lung Tissues. *Cancer Res.,* 2012, vol. 72, 3753-3763
- **KARP, P.H. et al.** An in vitro model of differentiated human airway epithelia. *Methods Mol. Biol.,* 2002, vol. 188, 115-137
- **MATHIS, C. et al.** Human bronchial epithelial cells exposed in vitro to cigarette smoke at the air-liquid interface resemble bronchial epithelium from human smokers. *American Journal of Physiology-Lung Cellular and Molecular Physiology,* 2013
- **MAUNDERS, H. et al.** Human bronchial epithelial cell transcriptome: gene expression changes following acute exposure to whole cigarette smoke in vitro. *Am J Physiol Lung Cell Mol Physiol.,* 2007, vol. 292, L1248-56
- **PEZZULO, A. A. et al.** The air-liquid interface and use of primary cell cultures are important to recapitulate the transcriptional profile of in vivo airway epithelia. *Am J Physiol Lung Cell Mol Physiol.,* 2011, vol. 300, L25-31
- **MATHERS, C.D. ; LONCAR, D.** Projections of global mortality and burden of disease from 2002 to 2030. *PLoS medicine,* 2006, vol. 3, e442
- **CHURG, A. ; COSIO, M. ; WRIGHT, J.L.** Mechanisms of cigarette smoke-induced COPD: insights from animal models. *American Journal of Physiology-Lung Cellular and Molecular Physiology,* 2008, vol. 294, L612-L631
- **PAUWELS, R.A. ; RABE, K.F.** Burden and clinical features of chronic obstructive pulmonary disease (COPD). *Lancet,* 2004, vol. 364, 613-20
- **RABE, K.F. et al.** Global strategy for the diagnosis, management, and prevention of chronic obstructive pulmonary disease: GOLD executive summary. *Am J Respir Crit Care Med.,* 2007, vol. 176, 532-55
- **BECKETT, E.L. et al.** A new short-term mouse model of chronic obstructive pulmonary disease identifies a role for mast cell tryptase in pathogenesis. *Journal of Allergy and Clinical Immunology,* 2013

- **WESTRA, J.W. et al.** Construction of a computable cell proliferation network focused on non-diseased lung cells. *BMC Syst Biol,* 2011, vol. 5, 105
- **SCHLAGE, W.K. et al.** A computable cellular stress network model for non-diseased pulmonary and cardiovascular tissue. *BMC Syst Biol,* 2011, vol. 5, 168
- **GEBEL, S. et al.** Construction of a Computable Network Model for DNA Damage, Autophagy, Cell Death, and Senescence. *Bioinformatics and Biology Insights,* 2013, vol. 7, 97-117
- **W. STINN, A. et al.** Lung inflammatory effects, tumorigenesis, and emphysema development in a long-term inhalation study with cigarette mainstream smoke in mice. *Toxicol. Sci.,* 2012, vol. 305, 49-64
- **WESTRA, J.W. et al.** Construction of a computable cell proliferation network focused on non-diseased lung cells. *BMC SystBiol,* 2011, vol. 5, 105